(19) Europäisches Patentamt
European Patent Office
Office européen des brevets

(11) EP 1 364 966 A2

(12) EUROPEAN PATENT APPLICATION

(43) Date of publication:
**26.11.2003 Bulletin 2003/48**

(51) Int Cl.7: **C07K 14/485**, C12N 15/18, C12N 15/82, A61K 38/18

(21) Application number: **03252728.5**

(22) Date of filing: **30.04.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **30.04.2002 US 377294 P**

(71) Applicant: **Alberta Research Council of Canada**
**Edmonton, Alberta T6N 1E4 (CA)**

(72) Inventors:
- **Kenward, Kimberly D.**
  **Vegreville, Alberta T9C 1B9 (CA)**
- **Shah, Salehuzzaman**
  **Edmonton, Alberta T6L 7G3 (CA)**

(74) Representative: **Main, Malcolm Charles**
**Murgitroyd & Company**
**Scotland House**
**165-169 Scotland Street**
**Glasgow G5 8PL (GB)**

# (54) Production of recombinant human epidermal growth factor in plants

(57) The present invention is directed to novel nucleic acid molecules that encode epidermal growth factor (EGF) protein. The EGF is optimized for expression in a plant. Vectors, genetic constructs, and transgenic plants comprising plant-optimized nucleotide sequences encoding EGF are disclosed. The nucleic acid molecules and corresponding vectors, and transgenic plants are useful for achieving large-scale or high-yield production of EGF.

**Figure 1A**
**AP.EGF.KII**

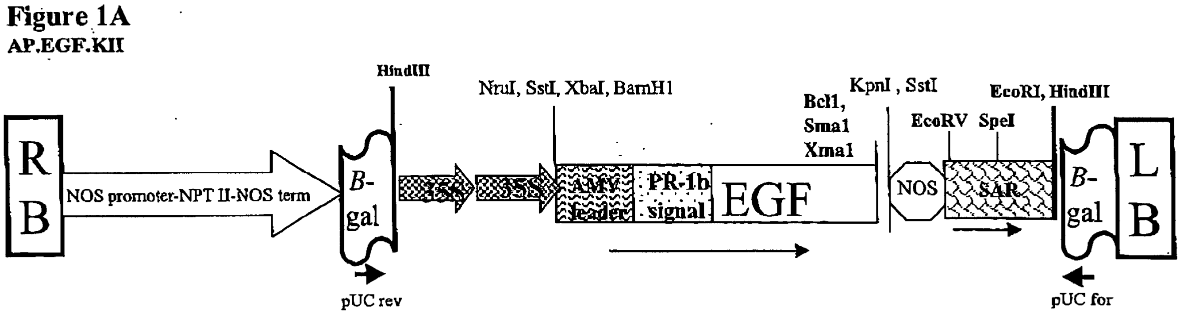


**Figure 1B**
**AP.EGF.KDEL.KII**

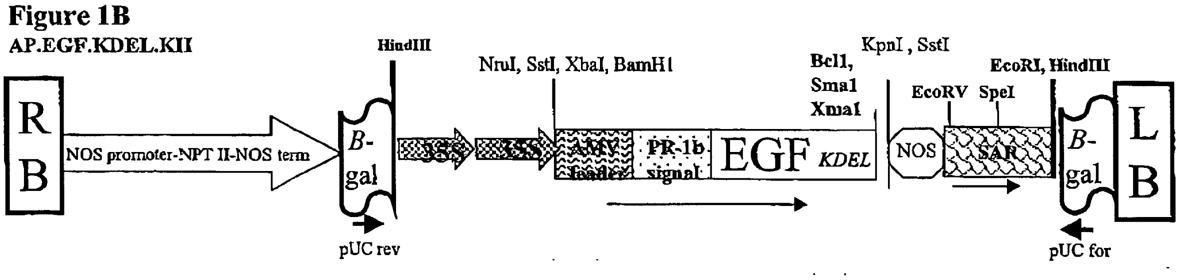


Figure 1

EP 1 364 966 A2

**Figure 1C**
**AP.EGF X**

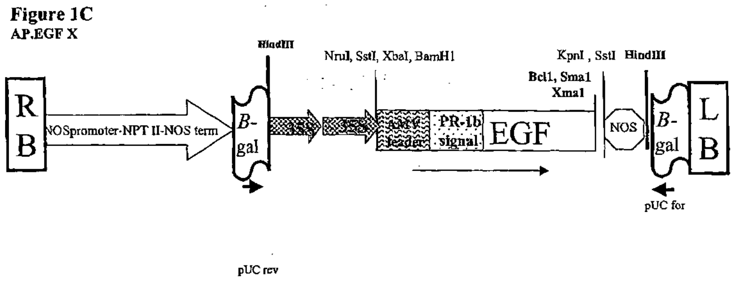


**Figure 1D**
**AP.EGF.KDEL X**

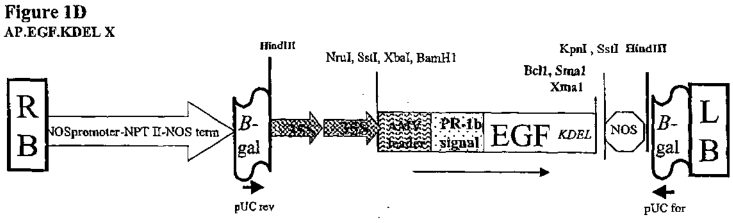


Figure 1 continued

**Figure 1E**
**AP.EGF KI**

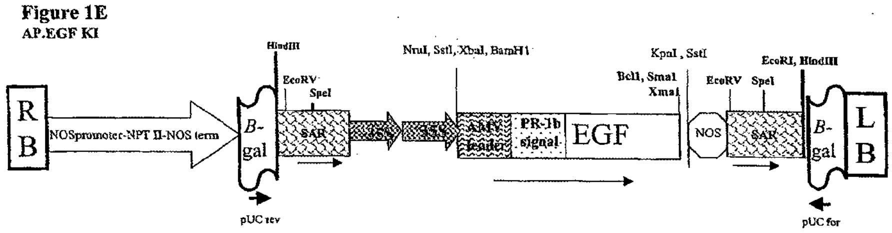


**Figure 1F**
**AP.EGF.KDEL KI**

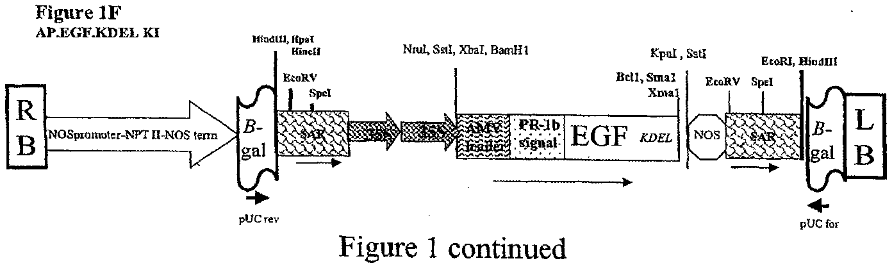


Figure 1 continued

**Figure 1G**
**AP.EGF KIII**

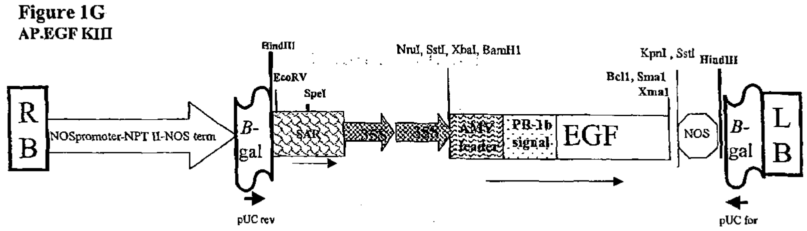


**Figure 1H**
**AP.EGF.KDEL KIII**

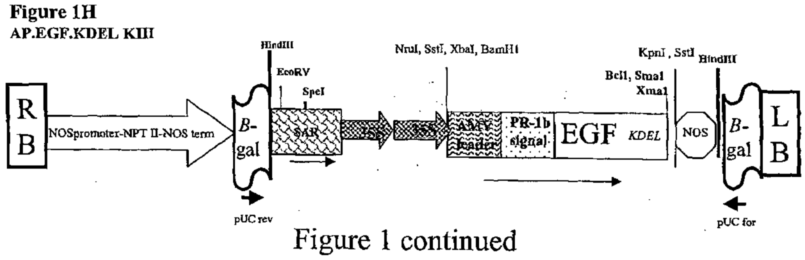


Figure 1 continued

## Description

The present invention relates to epidermal growth factor (EGF), and a method for producing EGF. More specifically, the invention relates to the production of EGF in plants.

## BACKGROUND OF THE INVENTION

Naturally occurring mature human EGF is a single-chain polypeptide comprised of 53 amino acids, of approximately 62 kDa.. It is produced in vivo as the processed product of a very large (1207 amino acids long) precursor protein, and is secreted in the saliva of ruminant and non-ruminant mammals, The precursor protein consists of a signal peptide, a large extracellular domain, a small transmembrane domain, and a cytoplasmic domain. The cxtracellular domain contains nine structurally homologous sub-domains which contain three disulfide bonds each and are considered characteristic of this protein. Within the EGF extracellular domain the EGF-like subdomains 2, 7 and 8 bind calcium. Nine N-glycosylation sites have been identified in the precursor molecule, all within the extracellular domain and none within the active EGF protein component. The active EGF peptide occurs at the C-terminal end of the extracellular domain just prior to the transmembrane domain, and encompasses all of EGF-like subdomain 9. The active EGF protein is thought to be removed from the membrane-bound precursor by a serine protease belonging to the kallikrein subfamily. Processing results in the release of the active 6.2 kDa EGF peptide and two 45 kDa peptides thought to represent the N-terminal extracellular domain, EGF from other mammalian sources varies from about 48 to about 53 amino acids in length.

EGF is a hormone that plays an important role in epithelial cell proliferation at early stages of development (Fisher and Lakshmanan, 1990, Endocrine Reviews 11(3):418-442). Native EGF is primarily associated with the gastrointestinal tract and is present in saliva, urine and the intestine. The protein is produced in the submaxillary gland, kidney, incisor tooth buds, lactating breast, pancreas, small intestine, ovary, spleen, lung, pituitary, and liver.

EGF has been associated with liver regeneration following injury, and gastrointestinal effects for better weight gain and decreased diarrhea. EGF also performs cytoprotective functions in the gastrointestinal tract, such as decreased gastric acid secretion, increased healing of ulcers, and increased crypt cell production rates after injury (Marti et al., 1989, Hepatology 9:126; Fisher and Lakshmanan, 1990, Endocrine Reviews 11(3):418-442).

US 5,218,093 discloses medicinal use of EGF for the treatment of soft-tissue wounds, and US 5,753,622 teaches of the use of EGF as a gastrointestinal therapeutic.

EGF is also known to promote new growth of epithelials cells (eg. skin, cornea, gastrointestinal tract, lungs) and may be used in wound healing, for example with burn patients, surface wounds and multi-organ failure, as a mucosal protectant from oral complications resulting from head and neck radio- or chemo-therapy (early evaluation stages), corneal (eye) wound healing, perforated tympanic membranes (ears), or lung injury. EGF may also be used within diabetes treatment, including complication healing (eg. foot ulcer), or pancreatic differentiation and growth.. Other uses include cosmetic skin care products, biological wool gathering from sheep, or as a veterinary food additive and gastrointestinal therapeutic agent, increased production in pigs and beef, and as a non-antibiotic method to control infection. EGF may also be used for treating premature organ development (*e.g*. intestine, lungs), or protection of liver from chemical poisoning.

Based on its potential industrial, cosmetic, nutritional, and pharmaceutical uses, there is a need for large-scale production of EGF. However, at present, there is no described method of producing a mature human EGF to a level sufficient for industrial application. Recombinant human proteins, including recombinant EGF, are known to be produced by expression and extraction from mammalian cell cultures. However, due to difficulties of protein purification from mammalian cells this process is slow and expensive.

US 5,652,120 relates to a process for expression and purification of recombinant human EGF from E. coli. However, the recombinant hEGF encoding sequence contains a methionine initiation codon, thereby producing an altered hEGF as compared to naturally occurring hEGF that does not have an N-terminal methionine. Moreover, synthesis methods using transformed bacterial strains are often expensive and have problems such as protein folding difficulties, inability to glycosylate proteins, and relegation of foreign peptides to insoluble material accumulated in inclusion bodies. Furthermore, the reducing environment of the bacterial cytosol is not well suited for production of proteins, such as EGF, that contain disulfide bonds. US 5,096,825 relates to expression of a recombinant human EGF in yeast cells. The hEGF produced in this system differs from naturally occurring hEGF in that it also contains an extra N-terminal methionine residue.

Plant-based production systems are a cheaper alternative to production of proteins in bacterial and yeast bioreactors, and can be used to generate large-scale amounts of protein that are properly folded and glycosylated. Transgenic tobacco plants have been used for the production of human EGF (Higo ct al., 1993, Biosci Biotechnol Biochem 57:1477-1481). However, the expression of the hEGF in the tobacco was unsatisfactory and produced negligible levels of protein (0.000006 % of total soluble protein; 20-60 pg/mg of total soluble protein) as determined by ELISA.

WO 98/21348 (Hooker et al.) discloses, transgenic tobacco plants that express a transgene encoding the 1207 amino acid precursor hEGF protein. Although the level of hEGF production is increased 10 to 70 fold in comparison to Higo et al., Western Blot analysis indicates that the expressed protein is 250 amino acids long, indicating a partially processed EGF protein. Further processing would be required to convert this protein into an active, mature hEGF protein of 53 amino acids. Furthermore, the yield of the partially processed protein, although greater than the yield disclosed by Higo et al., is still quite low (0.0004% of total soluble protein; 4.1 ng/mg of total soluble protein). This document also suggests a method to increase production rates of hEGF in transgenic plants by introducing a construct encoding a tetramer of hEGF units that are subsequently cleaved to provide hEGF. However, the method is complex and further processing of these tetramers is not disclosed.

Quanhong et al. (GenBank Accession AF284213), disclose a nucleotide sequence encoding a fusion of a plant PR-S signal peptide and a mature hEGF protein. The portion of the nucleotide sequence encoding the mature hEGF protein is optimized to account for codon usage in plants. However, no transgenic plants are disclosed, nor are any protein yields of hEGF in plants determined.

The present invention provides for increased levels of production of the mature EGF in plants and, for the delivery of an active and mature EGF using plant tissues.

It is an object of the invention to overcome disadvantages of the prior art.

The above object is met by the combinations of features of the main claims, the sub-claims disclose further advantageous embodiments of the invention.

## SUMMARY OF THE INVENTION

The present invention relates to epidermal growth factor (EGF), and a method for producing EGF. More specifically, the invention relates to the production of EGF in plants.

According to the present invention there is provided a nucleic acid molecule that encodes an epidermal growth factor protein (EGF) or a fragment thereof, the nucleic acid molecule also comprising a KDEL sequence, a scaffold attachment region (SAR), a nucleic acid sequence encoding an affinity tag, or a combination thereof, wherein the fragment of EGF exhibits EGF-biological activity. Preferably, the nuclcotide sequence has been optimized for expression in plants. More preferably the EGF is mammalian EGF.

The present invention includes the nucleic acid molecule as defined above wherein the EGF is selected from the group consisting of hEGF, pig EGF, rat EGF, mouse EGF, cat EGF, dog EGF and horse EGF. Preferably the EGF is human EGF or cat EGF.

The present invention also pertains to a nucleic acid molecule as defined above, wherein the hEGF is encoded by the nucleotide sequence defined by SEQ ID NO:3, an analogue, fragment, or derivative thereof, providing that the analogue, fragment, or derivative thereof encodes a product that exhibits EGF-biological activity, the analogue, fragment, or derivative thereof comprising at least about 61% homology with the nucleotide sequence defined by SEQ ID NO:3 as determined using BLAST, with the following parameters: Program: blastn; Database; nr; Expect 10; filter: low complexity; Alignment: pairwise; Word size: 11.

The present invention also embraces a nucleic acid molecule that encodes an epidermal growth factor protein (EGF) or a fragment thereof, the nucleic acid molecule also comprising a KDEL sequence, a scaffold attachment region (SAR), a nucleic acid sequence encoding an affinity tag, or a combination thereof, wherein the EGF is encoded by the nucleotide sequence defined by SEQ ID NO:3, an analogue, fragment, or derivative thereof, providing that the analogue, fragment, or derivative thereof encodes a product that exhibits EGF-biological activity, the analogue, fragment, or derivative thereof hybridizes to the hEGF under stringent conditions, the stringent conditions comprising, hybridization at 65°C overnight in 0.5 M sodium phosphate, 7% SDS, 10 mM EDTA, salmons sperm DNA, followed by washing, for 30 min each, at 65°C 2xSSC, 0.1% SDS, then 1xSSC, 0.1% SDS, and then 0.1SXSC, 0.1% SDS.

The present invention relates to a nuclcic acid molecule that encodes an epidermal growth factor protein (EGF) or a fragment thereof, the nucleic acid molecule also comprising a KDEL sequence, a scaffold attachment region (SAR), a nucleic acid sequence encoding an affinity tag, or a combination thereof, and further comprises at least one nucleotide sequence encoding a signal sequence peptide, the signal sequence peptide is obtained from a protein selected from the group consisting of a pathogenesis related protein, pathogenesis-related protein 1a, pathogenesis-related protein 1b, pathogenesis-related protein 1c, pathogenesis-related protein S, sporamin, extensin, potato proteinase inhibitor II, lectin, EGF, preproricin, human alpha-lattalbumin, and human alpha-lactoferrin.

The present invention pertains to a nucleic acid molecule that encodes an epidermal growth factor protein (EGF) or a fragment thereof, the nucleic acid molecule also comprising a KDEL sequence, a scaffold attachment region (SAR), a nucleic acid sequence encoding an affinity tag, or a combination thereof, wherein the SAR is obtained from the group consisting of a soybean, a tobacco, a tomato, Arabidopsis, and petunia.

The present invention also provides a vector comprising a nucleic acid molecule that encodes an epidermal growth factor protein (EGF) or a fragment thereof, the nucleic acid molecule also comprising a KDEL sequence, a scaffold attachment region (SAR), a nucleic acid sequence encoding an affinity tag, or a combination thereof, operatively linked with a regulatory region and terminator region.

Also provided by the present invention is a plant cell, plant seed, a plant, or progeny thereof, comprising the vector as just described.

The present invention pertains to a method of producing a transgenic plant that expresses an epidermal growth factor comprising;

i) introducing into a plant, a nucleic acid molecule that encodes an epidermal growth factor protein (EGF) or a fragment thereof, the nucleic acid molecule also comprising a KDEL sequence, a scaffold attachment region (SAR), a nucleic acid sequence encoding an affinity tag, or a combination thereof, to produce one or more transformed plants;
ii) selecting from the one or more transformed plants an EGF-expressing transformed plant; and
iii) growing the EGF-expressing transformed plant to produce the transgenic plant that expresses EGF.

The present invention also relates to a method of treating a mammal with an epidermal growth factor (EGF) comprising,

i) introducing into a plant, a nucleic acid molecule that encodes an epidermal growth factor protein (EGF) or a fragment thereof, the nucleic acid molecule also comprising a KDEL sequence, a scaffold attachment region (SAR), a nucleic acid sequence encoding an affinity tag, or a combination thereof, to produce one or more transformed plants;
ii) selecting from the one or more transformed plants an EGF-expressing transformed plant;
iii) growing the EGF-expressing transformed plant to produce a transgenic plant that expresses EGF;
iv) feeding the transgenic plant that expresses EGF to the mammal.

The present invention embraces a method for producing epidermal growth factor (EGF) comprising,

i) introducing into a plant, a nucleic acid molecule that encodes an epidermal growth factor protein (EGF) or a fragment thereof, the nucleic acid molecule also comprising a KDEL sequence, a scaffold attachment region (SAR), a nucleic acid sequence encoding an affinity tag, or a combination thereof, to produce one or more transformed plants;
ii) selecting from the one or more transformed plants an EGF-expressing transformed plant;
iii) growing the EGF-expressing transformed plant to produce a transgenic plant that expresses EGF;
iv) harvesting tissue from the transgenic plant that expresses EGF; and
v) extracting the EGF from the tissue.

Furthermore, following the step of extracting, the EGF may be purified.

The present invention also provides a method of producing an epidermal growth factor comprising, growing a plant that comprises a nucleic acid molecule that encodes an epidermal growth factor protein (EGF) or a fragment thereof, the nucleic acid molecule also comprising a KDEL sequence, a scaffold attachment region (SAR), a nucleic acid sequence encoding an affinity tag, or a combination thereof, operatively linked with a regulatory region and terminator region, to produce the EGF.

This summary of the invention does not necessarily describe all necessary features of the invention but that the invention may also reside in a sub-combination of the described features.

## BRIEF DESCRIPTION OF THE DRAWINGS

These and other features of the invention will become more apparent from the following description in which reference is made to the appended drawings wherein:

**FIGURE 1** shows several genetic constructs used for the production of hEGF in plants. **Figure 1A** shows the components of clone AP.EGF KII, comprising an AMV 5' untranslated region (AMV leader), a signal peptide (PR-1b signal), a plant optimized EGF, and a SAR sequence. **Figure 1B** shows the components of AP.EGF.KDEL KII, comprising AMV 5' untranslated region (AMV leader), signal peptide (PR-1b signal), a plant optimized EGF, a KDEL sequence, and a SAR sequence **Figure 1C** shows the components of AP.EGF.X, comprising an AMV 5' untranslated region (AMV leader), a signal peptide (PR-1b signal), a plant optimized EGF, and which lacks a KDEL and a SAR sequence. **Figure 1D** shows the components of AP.EGF.KDEL X, comprising comprising an AMV 5' untranslated region (AMV leader), a signal peplide (PR-1b signal), a plant optimized EGF, and a KDEL sequence, but lacks a SAR sequence. **Figure 1E** shows the components of clone AP.EGF KI, comprising a SAR sequence, an AMV 5' untranslated region (AMV leader), a signal peptide (PR-1b signal), a plant optimized EGP, and a SAR sequence. **Figure IF** shows the components of clone AP.EGF.KDEL KI, comprising a SAR sequence, an AMV 5' untranslated region (AMV leader), a signal peptide (PR-1b signal), a plant optimized EGF, a KDEL sequence, and a SAR sequence. **Figure 1G** shows the components of clone AP.EGF KIII, comprising a SAR sequence, an AMV 5' untranslated region (AMV leader), a signal peptide (PR-1b signal), and a plant optimized EGF. **Figure 1H** shows the components of clone AP.EGF.KDEL KIll, comprising a SAR sequence, an AMV 5' untranslated region (AMV leader), a signal peptide (PR-1b signal), a plant optimized EGF, and a KEEL sequence.

**FIGURE 2** shows a comparison of the sequences of mammalian, and plant optimized EGFs. **Figure 2A** shows a comparison of the sequence of various mammalian EGFs. Row (1): nucleotide sequence of human kidney hEGF (SEQ ID NO:2); Row (2): a low homology modified EGF (SEQ ID NO:12, where M= A or C; B= C, G, or T; H= A, C, or T; W= A or T; D= A, G, or T; V= A, C, or G); Row (3): an EGF optimized for tobacco plant production (100% optimized, all codons are optimized for plant expression; SEQ ID NO:11); Row (4): an EGF comprising least favoured codon use with respect to tobacco production (0% optimized); SEQ ID NO:13); Row (5): an optimized EGF as described in Example1 herein (SEQ ID NO:3); Row (AA): the hEGF amino acid sequence (SEQ ID NO:1), Row (CS): the consensus sequence for various EGF nucleotide sequences shown in Figure 2A. **Figure 2B** shows a comparison of modified EGF nucleotide sequence with the native human EGF nucleotide sequence. Row (1): the nucleotide sequence for native human EGF (SEQ ID NO:2); Row (2): nucleotide sequence for a modified EGF nucleotide sequence optimized for expression in plants as described in Example 1 (SEQ ID NO:3); Row (3): nucleotide sequence for a modified EGF nucleotide sequence optimized for expression in plants as described in Example 1 and comprising a KDEL sequence (SEQ ID NO:30); Row (AA): the amino acid sequence of EGF (SEQ ID NO:41); Row (CS): Row (CS): the consensus sequence for various EGF nucleotide sequences shown in Figure 2B. **Figure 2C** shows a comparison of the amino acid sequences of several mammalian EGF's including: "EGF": EGF encoded by nucleic acid seqeunce of SEQ ID NO:3, Human (Homo sapiens; NP_001954; SEQ ID NO:1), Pig (Sus scrofa; AF336151; SEQ ID NO:17), Rat (Rattus norvegicus; NP_036974; SEQ ID NO:18), Mouse (Mus musculus; NP_034243; SEQ ID NO:19), Cat (Felis catus; BAB47391; SEQ ID NO:20), Dog (Canis familiaris; BAB40599; SEQ ID NO:21), and Horse (Equus caballus; AAB32226; SEQ ID NO:22). The consensus sequence is also indicated at the bottom of the figure. **Figure 2D** shows an example of the sequence of Cat EGF, and variations of the Cat EGF sequence for plant expression. Row (1): nucleotide sequence encoding mature Cat EGF (SEQ ID NO:29); Row (2): nucleic acid sequence comprising most-favoured codons for EGF production in tobacco (100% optimized; SEQ ID NO: 23); Row (3): partially optimized coding sequence (uses 1-3rd choice codons to accommodate relative use of different codons in tobacco; SEQ ID NO:24); Row (4): un-optimized Cat EGF for production in tobacco (0% codon optimization using all least-favoured codons; SEQ ID NO:25); Row (5): nucleic acid sequence comprising most favoured codons for EGF production in canola (100% optimized; SEQ ID NO:26); Row (6): partially optimized coding sequence (uses 1-3rd choice codons to accommodate relative use of the different codons in canola; SEQ ID NO:27); Row (7): unoptimized Cat EGF for EGF production in canola (0% codon optimization using all least-favoured codons; SEQ ID NO:28); Row (AA): amino acid translation of Cat EGF (SEQ ID NO:20); Row(CS): the consensus sequence for various Cat EGF nucleotide sequences shown in Figure 2D.

**FIGURE 3** shows results for PCR analysis, to determine transgenic identity, of transformed plants comprising various constructs of the present invention. **Figure 3A** shows wild-type and transformed N. tabacum cv, Xanthi plants. **Figure 3B** shows transformed N. tabacum 81V-9 plants. Quality of the DNA extracts was determined by amplification of the native tobacco acetolactate synthase gene (lane 1 in each group). Transgenic plants were screened for the presence and orientation of the transgene construct elements from the CaMV 35S promoter through the NOS terminator (lane 2), AMV through EGF coding sequence (lane 3) and the EGF coding sequence through to the SAR (lane 4).

**FIGURE 4** shows Western blot detection of plant-produced EGF. **Figure 4A** shows AP.EGF X and AP.EGF.KDEL X transformants (no SAR present). **Figure 4B** AP.EGF KI and AP.EGF.KDEL KI transformants (comprising 5' and 3' SARs). **Figure 4C** AP.EGF KII and AP.EGF.KDEL KII transformants (3' SAR only). **Figure 4D** AP.EGF KIII and AP.EGF.KDEL KIII transformants (5' SAR only). Equivalent amounts of total soluble protein were loaded to allow direct comparison of AP.EGF and AP,EGF.KDEL production within a construct series. Different total amounts of protein were loaded between the X, KI, KII and Kill constructs to ensure a visible signal in each case (see Example 3).

**FIGURE 5** shows a comparison of EGF production in transgenic plants. **Figure 5A,** plants transformed with AP.

EGF.X. **Figure 5B,** plants transformed with AP.EGF.KDEL.X. **Figure 5C,** plants transformed with AP.EGF.K1. **Figure 5D,** plants transformed with AP.EGF.KDEL.K1.

## DESCRIPTION OF PREFERRED EMBODIMENT

The present invention pertains to method for optimizing production of a recombinant mature epidermal growth factor. More specifically, the invention relates to high-yield production of mature EGF in plants. Furthermore, the present invention pertains to the extraction of EGF from transgenic plants, or the administration of tissues of the transgenic plant for cosmetic, medicinal, veterinarial, industrial, or nutritional purposes.

The following description is of a preferred embodiment by way of example only and without limitation to the combination of features necessary for carrying the invention into effect.

The present invention provides an effective method for the reliable production of EGF, for example but not limited to mammalian EGF, human EGF (hEGF), or a modified EGF in plants. Prior art methods for the expression of recombinant hEGF in transgenic plants have resulted in very low yields. The hEGF produced by the method disclosed byHigo et al. (1993, Biosci Biotechnol.Biochem 57:1477-1481) results in hEGF production constituting 0.000006 % of total soluble protein. The method disclosed in WO98/21348 only achieves production of a partially processed hEGF protein at a level of 0.0004% of total soluble protein. Since mature hEGF only makes up one fourth of the partially processed hEGF, this method only produces mature hEGF at a level of 0.0001% of total soluble protein.

As described herein, increased expression of EGF in plant tissues may be obtained by utilizing a modified nucleotide sequence. These modified sequences may comprise, but are not limited to, an altered G/C content, for example, to more closely approach that typically found in plants, along with the removal of codons atypically found in plants. However, G/C content may be modified to assist in ensuring start and stop codon recognition (e.g. Angenon, G., et al., 1990, FEBS Lett. 271, 144-146). Furthermore, addition of introns, preferably towards the 5' region of a gene, or altering the context of start and stop codons may also result in increased expression or transcript stability, or both. Addition of Kozak's (Kozak., M., J.Mol. Biol. (1987) 196(4), 947-50) consensus or Lutcke's (Lutcke H.A., et al.: EMBO J. 1987 6(1) 43-8) consensus sequence to a gene may be used to help establish the correct start codon for translation Other modifications include alteration of premature poly-A signals, mRNA destabilizing sequences and intron-like sequences. Furthermore, strategies relating to targeting the protein encoded by a transgene to specific compartments within the cell, for example but not limited to the ER, can be adopted to address the problem of low levels of foreign protein expression in genetically transformed plants. Other organelles may also be targeted as required and may include targeting the transgene protein to the endoplasmic reticulum (ER), vacuole, apoplast, or chloroplast. Expression may also be increased through the use of translational fusions. For example, the transgene protein may be fused with a signal peptide that directs protein synthesis in plants into the desired cellular compartment, for example the ER. Optionally, the transgene fusion could comprise a second signal peptide that allows for retention of proteins in the ER or targeting of proteins to the vacuole. A non-limiting example of a signal sequence that may be used to target and retain the protein within the ER is the H/KDEL sequence (Schouten et al 1996, Plant Molec. Biol. 30, 781-793). Replacing any secretory signal sequence with a plant secretory signal may also ensure targeting to the endoplasmic reticulum (Denecke et al 1990, Plant Cell 2, 51-59). Furthermore, the EGF sequence may also be modified to include a scaffold attachment region (SAR) to aid in increased expression of the construct. Other sequences, to aid in the isolation and purification of the EGF protein, may also be introduced into the nucleotide sequence as disclosed herein, including, but not limited to, one or more affinity tags, for example but not limited to a HIS tag.

In an aspect of an embodiment, the method of the present invention relates to transforming a plant with a chimeric construct which comprises an EGF, a fragment, or a derivative thereof in a plant to produce a transformed plant. Preferably, the EGF is a mammalian EGF, for example, but not limited to human, pig, rat, mouse, dat, dog, or horse, EGF (Figure 2B). More preferably, the mammalian EGF is hEGF. More preferably still, the EGF is a modified mammalian EGF, or a modified hEGF (e.g. Figures 2A, 2B, and 2D). Therefore the present invention includes plants, plant cells or plant seeds comprising a nucleotide sequence which encode EGF, a fragment or a derivative thereof.

The protein produced by the method of the present invention may comprise full-length mature (of approx. 6.2 kDa) EGF, for example but not limited to SEQ ID NO:1, or a fragment or derivative thereof, for example SEQ ID NO: 41 (EGF+KDEL). As shown in Figure 2C, mammalian EGF varies from about 48 to about 53 nucleotides in length. As will be appreciated by someone of skill in the art, an entire protein may not be required for the biological efficacy of EGF within a mammal, but rather, it may be possible that a smaller fragment of the protein can be used. Preferably the form of EGF produced by the plant is full-length mature (of approx.6.2 kDa) EGF protein having about 48 to about 53 amino acids. However, the actual length of the amino acid sequence may vary depending upon the source of the EGF, the signal sequence, ER retention sequence, or protein purification tag sequence that may be added to the EGF sequence (e.g. see Figures 1A-H). More than one of these additional sequences may be added to the EGF sequence. Furthermore, these additional sequences may be repeated if desired. A protein may retain biological activity even with additional protein segments attached, so a larger variant of the protein may also be used. Added segments could include signal peptides, targeting signals (eg. KDEL), protein purification tags or other fusion protein components. A non-limiting example of a mammalian EGF optimized for plant expression and comprising a KDEL sequence is provided in Figure 2B (SEQ ID NO:30).

The protein produced by the method of the present invention may be partially or completely purified from the plant. In addition, the protein may be formulated into a form for topical application (e.g. cosmetic use), oral use or an injectable dosage form. Furthermore, the protein produced by the method of the present invention may be used for administration to a mammal.

The protein produced by the method of the present invention, which comprises EGF and fragments thereof may have a variety of uses including, but not limited to the production of biologically active proteins for use as oral proteins, for systemic administration, for general research purposes, or combinations thereof. Further, the protein produced by the method of the present invention may be produced in large quantities in plants, isolated and optionally purified at potentially reduced costs compared to other conventional methods of producing proteins such as but not limited to those which employ fermentation processes.

In order to optimize the expression of a foreign gene within plants, the EGF gene may be modified or altered from its naturally occurring nucleotide sequence as required so that the corresponding protein encoded by the modified gene is produced at a level higher than the protein encoded by the naturally-occurring or native gene. Preferably the modified EGF nucleotide sequence is optimized for codon usage, GC content, or both codon usage and GC content within a plant, and demonstrates at least about 60.5% identity with the naturally occurring EGF nucleotide sequence. For example, without wishing to be limiting, Figure 2B shows a nucleotide sequence alignment of a modified EGF nucleotide sequence of the present invention (SEQ ID NO:3), with a naturally-occurring or native EGF nucleotide sequence (SEQ ID NO:2), where the modified EGF nucleotide sequence is 75.9% identical with the naturally-occurring EGF nucleotide sequence. It is preferred that the proteins encoded by the modified EGF nucleotide sequence and the naturally-occurring EGF nucleotide sequence are 100% identical with respect to amino acid sequence.

It is to be understood that 51 of the 54 codons encoding mature EGF may be modified without altering the final amino acid sequence (SEQ ID NO:1) of EGF in order to optimize expression of EGF in a plant. For example, with reference to Figure 2A, there is shown a most-favoured plant optimized EGF sequence (SEQ ID NO:11; row (3) of Figure 2A) that exhibits 78.4% identity with hEGF. A low homology EGF sequence (SEQ ID NO:13) that exhibits 60.5% identity, yet still encodes hEGF (SEQ ID NO.1) is also shown in Figure 2A, row (4), as is a modified EGF comprising multiple codon options for plant expression (one example of possible degenerate sequences encoding EGF; SEQ ID NO:12; row (2)). Table 1 shows a comparison of EGF sequence identities for various native and modified EGF sequences depicted in Figures 2A, 2B and 2D.

Table 1:

| **Comparison of various EGF sequences to native hEGF or Cat EGF (see Figures 2A, 2b and 2D).** | | |
|---|---|---|
| **Relative to hEGF** | **Sequence ref** | **Identity** |
| hEGF | SEQ ID NO:2 | 100% |
| tobacco optimized hEGF | SEQ ED NO:3 | 75.9% |
| tobacco optimized hEGF plus KDEL | SEQ ID NO:30 | 75.9% |
| low homology hEGF* | SEQ ID NO:12 | 60.5% |
| hEGF100% optimized for tobacco** | SEQ ID NO: 11 | 78.4% |
| hEGF 0% optimized for tobacco*** | SEQ ID NO:13 | 75.9% |
| hEGF consensus sequence | SEQ ID NO:39 | |
| **Relative to Cat EGF** | | |
| Cat EGF | SEQ ID NO:29 | 100% |
| cat EGF 100% optimized for tobacco | SEQ ID NO:23 | 76.3% |
| partially optimized cat EGF[a] | SEQ ID NO:24 | 71.8% |
| cat EHG 0% optimized for tobacco | SEQ ID NO:25 | 78.2% |
| cat EGF 100% optimized for canola | SEQ ID NO:26 | 75.6% |
| partially optimized for canolaa | SEQ ID NO:27 | 77.6% |
| cat EGF 0% optimized for canola | SEQ ID NO:28 | 76.9% |

*low homology sequence refers to a one of several possible degenerate nucleotide sequences encoding EGF.

**100% optimized for expression in tobacco: nucleotide sequence wholly comprised of the most favoured codon for each ammo acid.

***0% optimized for plant expression, nucleotide sequence comprises all least favoured codons for plant expression.

[a]partially optimized: coding sequence that comprises first to third choices to accomodate relative use of the different codons in a plant.

Table 1: (continued)

| Comparison of various EGF sequences to native hEGF or Cat EGF (see Figures 2A, 2b and 2D). | | |
|---|---|---|
| **Relative to hEGF** | **Sequence ref** | **Identity** |
| cat EGF consensus sequence | SEQ ID NO:40 | |

Percentage of identity between EGF nucleotide sequences may be readily determined using sequence comparison techniques for example but not limited to a BLAST (available through GenBank URL: www.ncbi.nlm.nih.gov/cgi-bin/BLAST/, using default parameters, including: Program: blastn; Database: nr; Expect 10; filter: low complexity ; Alignment: pairwise; Word size:11) or FASTA, using default parameters.

The present invention includes nucleic acid sequences that encode EGF that may be modified as described herein. Preferably the EGF is mammalian EGF. Examples of mammalian EGF's that may be produced according to the present invention, and that are not to be considered limiting in any manner, are shown in Figure 2C, and include human EGF (SEQ ID NO:1), pig EGF (SEQ ID NO:17), rat EGF (SEQ ID NO: 18), mouse (SEQ ID NO:19), cat EGF (SEQ ID NO:20), dog EGF (SEQ ID NO:21), and horse EGF (SEQ ID NO:22). The amino acid sequences exhibit from about 62% identity with human EGF (horse EGF) to about 84.9% identity for pig EGF as determined using BLAST, set at default parameters (data base: nr, low complexity filter; expect 10; word size:3; matrix: BLOSUM62, gap costs: Existence:11, Extension:1).

It is also contemplated that fragments or portions of mature EGF or derivatives thereof, that exhibit useful biological properties (EGF-biological activities), may be expressed within plant tissues. Preferably, modified EGF, fragments, portions of mature EGF, or derivatives thereof, exhibit properties with respect to cosmetic, industrial, medical, veterinarial, or nutritional applications that are similar to those observed with the administration of native EGF. If required, further processing of the plant produced EGF may also be performed in order that the EGF exhibit a desired biological activity, for example, protein re-folding through chemical intervention.

EGF-biological activities include the detection of EGF via an antibody, for example in ELISA or Western analysis, the role EGF plays in the development of the oral cavity, lungs, gastrointestinal tract and eyelids, and the role that it may have in modulating development of the central nervous system (CNS) in fetal and neonatal mammals. Luminal EGF has been shown to increase cell proliferation in the gastrointestinal tract in a dose-dependent manner but the effect diminishes with increasing cell differentiation, In adult mice, EGF appears to inhibit acid secretion from the parietal cells of the stomach, play a role in wound healing (eg. ulcer), and has been shown to stimulate proliferation and differentiation of cells associated with the subependyma of the forebrain and tentatively identified as CNS stem cells. EGF also seems to be a key factor in initiating liver regeneration after partial hepatectomy or chemical injury: During liver regeneration the normal pathway to lysosomal degradation is shut down and EGF is diverted to the nucleus prior to initiation of DNA synthesis. Within the gastrointestinal tract EGF has been associated with diffuse lengthening of the brush border microvilli. Secondary effects of EGF include increased nutritional uptake and decreased bacterial colonization ofthe small and large intestines (resulting in better weight gain and decreased diarrhea). Plant-derived EGF may also be used for wound healing applications, treatment of premature organ development, reducing inflammation and cell damage in multiorgan failure, or in industrial applications in animal production, or as a cosmetic as an anti-aging skin rejuvenation treatment.

Therefore, the present invention relates to the production, within a plant, of a modified EGF, or a fragment or derivative thereof that retains one or more of the above EGF-biological properties, for example as shown in Figures 4 (Western analysis) and 5 (ELISA analysis).

The present invention also pertains to other modifications of the naturally occurring EGF gene, or to an EGF gene comprising an altered G/C or codon content, as described above, to optimize expression of the gene, stability and purification of the protein, or a combination thereof. For example, modification of the 5' or 3' region of natural or modified EGF genes can be carried out in order to enhance expression of the gene and target the product to an appropriate intercellular compartment to ensure stability.

An example of a 5' modification may include a signal peptide (signal sequence) to direct the protein to a specific cellular compartment, for example which is not to be considered limiting in any manner, the signal sequence from a tobacco pathogenesis related protein (Cornelissen et al. 1986, EMBO J. 5, 37-40; Genbank accession #X03465 (nt 30-131), which is incorporated herein by reference). Other non-limiting examples of heterologous signal peptides are: sweet potato sporamin signal peptide for production of human lactoferrin (Salmon et al., 1998, Prot. Expr. Purif. 13 (1) 127-35, which is incorporated herein by reference); *Nicotiana plumbaginifolia* extensin signal peptide characterized for use with NPT II reporter protein secretion from tobacco protoplasts (De Loose et al., 1991, Gene 99 (1) 95-100 , which is incorporated herein by reference); tobacco (*Nicotiana tabacum*) pathogen related protein S signal peptide for production of *Aspergillus niger* phytase in transgenic tobacco (Verwoerd et al., 1995, Plant Physiol. 109 (4) 1199-205 , which is incorporated herein by reference); potato proteinase inhibitor II signal peptide used to express yeast invertase in transgenic tobacco (Barrieu and Chrispeels, 1999, Plant Physiol. 120, 961-968 , which is incorporated herein by reference); and *Phaseolus vulgaris* lectin signal peptide for expression of E. coli 4-hydroxybenzoate: polyprenyldiphosphate 3-polyprenyltransferase (Boehm et al., 2000, Transgenic Res, 9(6) 477-86, which is incorporated herein by reference). Native signal peptides can also be used. For example, the native EGF (WO98/21348, which is incorporated herein by reference), native preproricin (Sehnke et al., 1999, Prot.Expr. Purif. 15(2) 188-95, which is incorporated herein by reference), native human alpha-lactalbumin. (Takase and Hagiwara, 1998, J.Biochem 123(3) 440-4, which is incorporated herein by reference), and native human lactoferrin (Salmon et al., 1998, Prot. Expr. Purif. 13 (1) 127-35, which is incorporated herein by reference) signal peptides have all been used in expression studies. As shown in Figures 1E and F, another 5' modification may include a scaffold attachment region (SAR).

A non-limiting example of a 3' modification includes a SAR that can be used to reduce variation in levels of gene expression that may be associated with the position of transgene insertion within the genome of a plant. However, other alterations to the 5', 3', regions, in addition to those listed above; or modifications within the coding sequence, for example, KDEL motifs, affinity tags protease cleavage sites and the like, may be utilized in order to optimize expression, stability and, optionally, purification of the expressed protein.

A SAR (which has also been referred to as matrix attachment regions or MAR) may be present in untranscribed regions at varying distances upstream or downstream of gene, or it may be located within an intron. SARs range in size from 300bp-2kB, and generally map to A+T rich regions. SARs demonstrate little sequence homology, and are therefore usually characterized by the presence of particular DNA motifs, including: A-box (AATAAA(A/C)AAA; SEQ ID NO:35) which has been proposed to cause DNA bending; T-box (TT(T/A)TATT(T/A)TT; SEQ ID NO:36) which has been proposed to discourage nucleosome formation; ATATTT motif proposed to generate stable base-unpaired structures which may act as a nucleation site for local unwinding of DNA;
GTN(A/T)A(T/C)ATTNATNN(G/A; SEQ ID NO:37), a consensus cleavage site for *Drosophila topoisomerase* II, overrepresented in yeast and animal SARs but not as common in plant SARs. As there is no universal or strictly conserved SAR sequence or motif, nuclear scaffold components are thought to recognize and bind a DNA structure rather than a specific sequence.

The following classification scheme for SARs has been proposed, with classes being distinguished based on the location of a SAR with respect to native gene sequences:

i) Structural/Loop boundary SARs: without wishing to be bound by theory, these SARs may serve as the bases of the chromatin loops, and they may bind to the scaffold with high affinity so that they are constitutively attached during entire cell cycle;
ii) Functional/Upstream Regulatory SARs: without wishing to be bound by theory, these SARs are present in close proximity to regulatory elements suggesting that they may bring sequences into close proximity to the scaffold, thereby facilitating interaction of promoter and enhancer elements with trans-acting and/or transcription factors which assemble on the nuclear scaffold. These SARs may also bind cell-type specific proteins of the matrix with less affinity in a transient, transcription-related manner; and
iii) Replication origin SARs.

Any SAR can be incorporated into the present invention, including, but not limited to, SARs that have been isolated from: soybean (Schoffl et al. 1993, Transgenic Research 2:93-100, Genbank accession #M11317 (nt: 1310-1710), which is incorporated herein by reference); tobacco (Allen et al., 1996, Plant Cell 8:899-913; US 5,773,695, which is incorporated herein by reference); tomato (MAR Chinn ct al. 1996, Plant Molec. Biol. 32: 959-68, which is incorporated herein by reference); petunia (Galliano et al., 1995, Mol. Gen. Genet. 247: 614-22 , which is incorporated herein by reference); and Arabidopsis (MAR; Liu et al. 1998, Plant Cell Physiol. 39:115-123 , which is incorporated herein by reference). SARs have also been characterized in yeast (Newlon and Theis, 1993, Curr. Opin. Genet. Dev. 3: 752-8; Allen et al., 1993, Plant Cell 5:603-13, which is incorporated herein by reference).

Without wishing to be bound by theory, modifying a transgene to incorporate a SAR may remove a position effect on transgene insertion and normalize gene expression per transgene copy by reducing gene silencing, limiting condensation of chromatin structure, or decreasing influence of cis-regulatory elements from neighbouring DNA. While, the use of soybean SAR (Genbank accession # M11317 (nucleotides 1310-1710), which is incorporated herein by reference) may be preferred due to its smaller size, other SARs may also be used to enhance transgene expression,

It is preferred that the synthetic gene encoding the mature protein comprises a codon bias similar to that found in genes that are highly expressed in plants. If desired, the modified EGF may also comprise a sequence that allows for extraction and purification of the EGF. For example which is not to be considered limiting an affinity tag, such as but not limited to a His-tag as is known in the art may be linked to the EGF protein. The affinity-tag of the protein may be used for the purification of the protein using chromatography, for example a $Ni^{2+}$ column may be used for the purification of HIS-tag containing proteins. If desired a cleavage site may also be introduced into the sequence so that the affinity-tag portion of the protein may be cleaved following purification. The cleavage site may be acted upon via sequence specific proteases as known within the art, or it may be cleaved in the presence of a chemical, as would be evident to one of skill in the art. It is also contemplated that the protein may be modified so that the protein is targeted to a compartment of the cell to enhance stability of the product, for example the plastid, mitochondria, or the lumen of the endoplasmic reticulum (ER). However, other sites may also be targeted for example, extracellular secretion, in order to simplify extraction protocols.

By "codon optimization" it is meant the selection of appropriate DNA nucleotides for use within a structural gene or fragment thereof that approaches codon usage within a plant. Therefore, an optimized gene or nucleic acid sequence refers to a gene in which the nucleotide sequence of a native or naturally occurring gene has been modified in order to utilize statistically-preferred or statistically-favored codons within a plant. Any method may be used to determine a nucleotide sequence that favours plant expression. The nucleotide sequence typically is examined at the DNA level and the coding region optimized for expression in plants determined using any suitable procedure, for example as described in Sardana et al. (1996, Plant Cell Reports 15:677-681). In this method, the standard deviation of codon usage (SDCU), a measure of codon usage bias, may be calculated by first finding the squared proportional deviation of usage of each codon of the native EGF gene relative to that of highly expressed plant genes, followed by a calculation of the average squared deviation. The formula used is:

$$SDCU = \sum_{n=1}^{N} [(X_n - Y_n)/Y_n]^2/N$$

Where $X_n$ refers to the frequency of usage of codon n in highly expressed plant genes, where $Y_n$ to the frequency of usage of codon n in the gene of interest and N refers to the total number of codons in the gene of interest. A table of codon usage from highly expressed genes of dicotyledonous plants is compiled using the data of Murray et al. (1989, Nuc Acids Res. 17:477-498).

Another example of a method of codon optimization is based on the direct use, without performing any extra statistical calculations, of codon optimization tables such as those provided on-line at the Codon Usage Database through the NIAS (National Institute of Agrobiological Sciences) DNA bank in Japan (http//www.kazusa.or.jp/codon/). The Codon Usage Database contains codon usage tables for a number of different species, with each codon usage table having been statistically determined based on the data present in Genbank. For example, the following table may be used for codon optimization of transgenes that are to be expressed in tobacco plants: (kazusa.or.jp/codon/cgi-bin/showcodon.cgi?species=Nicotiana+tabacum+[gbpln])

***Nicotiana tabacum*** **[gbpln]: 794 CDS's (281365 codons)**

**fields: [triplet] [frequency: per thousand] ([number])**

UUU 24.1 (6778) UCU 20.3 (5718) UAU 17.7 (4985) UGU 10.2 (2877)<br>
UUC 17.8 (5016) UCC 10.5 (2954) UAC 13.6 (3840) UGC 8.1 (2280)<br>
UUA 11.9 (3361) UCA 17.2 (4826) UAA 1.2 (351) UGA 1.1 (312)<br>
UUG 21.9 (6168) UCG 5.1 (1442) UAG 0.5 (150) UGG 11.3 (3185)

CUU 24.2 (6818) CCU 19.5 (5480) CAU 13.0 (3662) CGU 7.7 (2180)<br>
CUC 12.6 (3536) CCC 7.0 (1969) CAC 8.9 (2512) CGC 4.0 (1130)<br>
CUA 8.9 (2510) CCA 20.5 (5762) CAA 21.2 (5968) CGA 5.2 (1477)<br>
CUG 10.5 (2952) CCG 4.7 (1335) CAG 15.4 (4333) CGG 3.7 (1028)

AUU 28.0 (7865) ACU 21.5 (6054) AAU 27.2 (7662) AGU 12.7 (3578)<br>
AUC 14.0 (3951) ACC 10.0 (2809) AAC 18.8 (5290) AGC 10.1 (2831)<br>
AUA 12.9 (3619) ACA 17.0 (4771) AAA 30.6 (8618) AGA 15.1 (4248)<br>
AUG 24.2 (6815) ACG 4.4 (1248) AAG 33.7 (9489) AGG 12.4 (3489)

GUU 27.6 (7777) GCU 32.9 (9260) GAU 35.6 (0022) GGU 24.2 (6799)<br>
GUC 11.5 (3229) GCC 12.9 (3629) GAC 16.9 (4764) GGC 11.9 (3351)

GUA 11.1 (3125) GCA 22.9 (6439) GAA 34.1 (9586) QGA 24.0 (6762)
GUG 16.9 (4766) GCG 5.8 (1644) GAG 28.6 (8036) GGG 10.5 (2944)
Coding GC 43.92% 1st letter GC 51.46% 2nd letter GC 40.45% 3rd letter GC 39.85%

By using the above table to determine the most preferred or most favored codon(s) for each amino acid in a tobacco plant, a naturally-occurring nucleotide sequence encoding a protein of interest can be codon optimized for expression in tobacco by replacing codons that may have a low statistical incidence in the tobacco genome with corresponding codons, in regard to an amino acid, that are statistically more favored. However, less-favored codons may be selected to delete existing restriction sites, to create new ones at potentially useful junctions (5' and 3' ends to add signal peptide or termination cassettes, internal sites that might be used to cut and splice segments together to produce a correct full-length sequence), alter GC content, or to eliminate nucleotide sequences that may negatively effect mRNA stability or expression. A similar process may be repeated for any pant genome and appropriate nucleotide sequences derived. An example.of a mammalian EGF optimized for expression within canola is provided in Figure 2D (SEQ ID NO:26).

The naturally occurring or native EGF, for example but not limited to cat or human EGF gene may already, in advance of any modification, contain a number of codons that correspond to a statistically-favored codon in a particular plant species. Therefore, codon optimization of the native EGF nucleotide sequence, may comprise determining which codons, within the native EGF nucleotide sequence, are not statistically-favored with regards to a particular plant, and modifying these codons in accordance with a codon usage table of the particular plant. The modified nucleotide sequence of EGF, for example but not limited to a cat or human EGF gene may be comprised, 100 percent, of plant preferred codon sequences, while encoding a polypeptide with the same amino acid sequence as that produced by the native cat or human EGF gene. Alternatively, the modified nucleotide sequence of the EGF gene may only be partially comprised of plant preferred codon sequences with remaining codons retaining nucleotide sequences derived from the native cat or human EGF gene. A modified nucleotide sequence may be fully or partially optimized for plant codon usage provided that the protein encoded by the modified nucleotide sequence is produced at a level higher than the protein encoded by the corresponding naturally occurring or native gene. Preferably the modified EGF comprises from about 60.5% to about 100% codons optimized for plant expression. More preferably, the modified EGF comprises from 70% to 100% of codons optimized for plant expression. It is to be understood that any mammalian EGF may be modified as defined herein, and that the examples pertaining to human EGF (e.g Figures 2A and 2B) or cat EGF (Figure 2D) are not to be considered limiting in any manner.

A modified nucleotide sequence that is optimized for codon usage in a plant may possess a GC content that is similar to the GC content of nucleotide sequences that occur naturally and are expressed in that plant. However, the nucleotide sequence of a modified gene that has only been partially optimized for codon usage in a plant, may be further modified so as to approach the GC content of nucleic acid sequences that occur naturally and are expressed in that plant. For example, a modified human EGF gene, that is only partially optimized for codon usage in tobacco, may be further modified so as to approach the GC content of tobacco nucleotide sequences, while encoding a polypeptide with the same amino acid sequence as that produced by the native human EGF gene. Furthermore, a native or naturally occurring gene could be optimized with respect to GC content without considering codon optimization. The modified nucleotide sequence of the present invention may be additionally optimized to create or eliminate restriction sites, or to eliminate potentially deleterious processing sites, such as potential polyadenylation sites or intron recognition sites, or mRNA destabilizing sequences. In the non-limiting example provided in Figure 2B, 35 of the 54 codons were changed, with 24 changes to a more preferred codon, 10 neutral changes to break up restriction sites or potential hairpin-loop structures, and to introduce desired restriction sites.

By "gene", it is meant a particular sequence of nucleotides including the coding region, or fragment thereof, and optionally the promoter and terminator regions which regulates expression of the gene, as well as other sites required for gene expression for example a polyadenylation signal which regulates the termination of transcription. By "coding region" or "structural gene", it is meant any region of DNA that determines the primary structure of a polypeptide following genetic transcription and translation. Furthermore, fragments comprising regions of interest of a coding region or structural gene may also be employed as needed.

By "modified gene" it is meant a DNA sequence of a structural gene that is synthesized using methods known in the art for example but not limited to chemical syntheses, site directed mutagenesis, or PCR and related techniques. A modified gene can comprise a fragment or the entire coding region of a gene, for example, EGF. Furthermore, a modified gene may also comprise regulatory elements that enhance expression of the gene, such as a scaffold attachment region, enhancers, promoters, or terminators, or motifs that aid in the stability or cellular targeting of the protein product. It is also contemplated that a modified gene optionally includes regions useful for the isolation and purification of the protein, or the protein fragment, encoded by the synthetic gene such as an affinity-tag.

By "regulatory region" it is meant a nucleic acid sequence that has the property of controlling the expression of a nucleotide sequence, either DNA or RNA that is operably linked with the regulatory region. By "operatively linked" it is meant that the particular sequences interact either directly or indirectly to carry out their intended function, such as mediation or modulation of gene expression. The interaction of operatively linked sequences may for example be mediated by proteins that in turn interact with the sequences. For example, a transcriptional regulatory region and a sequence of interest are operably linked when the sequences are functionally connected so as to permit transcription of the sequence of interest to be mediated or modulated by the transcriptional regulatory region. Regulatory region typically refers to a sequence of DNA, usually, but not always, upstream (5') to the coding sequence of a structural gene, which controls the expression of the coding region by providing the recognition for RNA polymerase and/or other factors required for transcription to start at a particular site. However, it is to be understood that other nucleotide sequences, located within introns, or 3' of the sequence may also contribute to the regulation of expression of a coding region of interest. An example of a regulatory element that provides for the recognition for RNA polymerase or other transcriptional factors to ensure initiation at a particular site is a promoter element. A promoter element comprises a basal promoter element, responsible for the initiation of transcription, as well as other regulatory elements (as listed above) that modify gene expression.

Suitable regulatory regions may be derived from a variety of sources, including bacterial, fungal, or viral genes (see Goeddel (Gene Expression Technology: Methods in Enzymology 185, Academic Press, San Diego, CA., 1990, which is incorporated herein by reference). Examples of such regulatory sequences include, but are not limited to: a transcriptional promoter, enhancer, or RNA polymerase binding sequence, a ribosomal binding sequence, including a translation initiation signal. Additionally, depending on the vector employed, other sequences, such as an origin of replication, and sequences conferring inducibihty of transcription may be incorporated as required. It will also be appreciated that the necessary regulatory sequences may be supplied by the nucleotide sequence encoding the native protein and/or its flanking regions.

By "promoter" it is meant the nucleotide sequences at the 5' end of a coding region, or fragment thereof that contain all the signals essential for the initiation of transcription and for the regulation of the rate of transcription. The promoters used to exemplify the present invention, which are not to be considered limiting in any manner, are constitutive promoters that are known to those of skill in the art. However, if tissue specific expression of the gene is desired, for example seed, or leaf specific expression, then promoters specific to these tissues may also be employed. Furthermore, as would be known to those of skill in the art, inducible promoters may also be used in order to regulate the expression of the gene following the induction of expression by providing the appropriate stimulus for inducing expression. In the absence of an inducer the DNA sequences or genes will not be transcribed. Typically the protein factor that binds specifically to an inducible promoter to activate transcription is present in an inactive form that is then directly or indirectly converted to the active form by the inducer. The inducer can be a chemical agent such as a protein, metabolitc, growth regulator, herbicide or phenolic compound or a physiological stress imposed directly by heat, cold, salt, or toxic elements or indirectly through the action of a pathogen or disease agent such as a virus. A plant cell containing an inducible promoter may be exposed to an inducer by externally applying the inducer to the cell or plant such as by spraying, watering, heating or similar methods.

By "constitutive promoter" it is meant a regulatory element directs the expression of a gene throughout the various parts of a plant and continuously throughout plant development. Examples of known constitutive regulatory elements include promoters associated with the CaMV 35S transcript (Odell et al., 1985, Nature, 313: 810-812), the double cauliflower mosaic virus promoter, 2x35S (Kay et al., 1987, Science 236:1299-1302), the rice actin 1 (Zhang et al, 1991, Plant Cell, 3: 1155-1165) and triosephosphate isomerase 1 (Xu et al, 1994, Plant Physiol. 106: 459-467) genes, the maize ubiquitin 1 gene (Comejo et al, 1993, Plant Mol. Biol. 29: 637-646), the *Arabidopsis ubiquitin* 1 and 6 genes (Holtorf et al, 1995, Plant Mol. Biol. 29: 637-646), tobacco t-CUP promoter (WO/99/67389; US 5,824,872), and the tobacco translational initiation factor 4A gene (Mandel et al, 1995 Plant Mol. Biol. 29: 995-1004). The term "constitutive" as used herein does not necessarily indicate that a gene under control of the constitutive regulatory element is expressed at the same level in all cell types, but that the gene is expressed in a wide range of cell types even though variation in abundance is often observed.

The chirneric gene constructs of the present invention can further comprise a 3' untranslated (or terminator) region. A 3' untranslated region refers to that portion of a gene comprising a DNA segment that contains a polyadenylation signal and any other regulatory signals capable of effecting mRNA processing or gene expression. The polyadenylation signal is usually characterized by effecting the addition of polyadenylic acid tracks to the 3' end of the mRNA precursor. Polyadenylation signals are commonly recognized by the presence of homology to the canonical form 5' AATAAA-3' although variations are not uncommon.

Examples of suitable 3' regions are the 3' transcribed non-translated regions containing a polyadenylation signal of *Agrobacterium* tumour inducing (Ti) plasmid genes, such as the nopaline synthase (Nos gene) and plant genes such as the soybean storage protein genes and the small subunit of the ribulose-1, 5-bisphosphate carboxylase (ssRUBISCO) gene.

The gene constructs of the present invention can also include further enhancers, either translation or transcription enhancers, as may be required. These enhancer regions are well known to persons skilled in the art, and can include the ATG initiation codon and adjacent sequences. The initiation codon must be in phase with the reading frame of the coding sequence to ensure translation of the entire sequence. The translation control signals and initiation codons can be from a variety of origins, both natural and synthetic. Translational initiation regions may be provided from the source of the transcriptional initiation region, or from the structural gene, The sequence can also be derived from the promoter selected to express the gene, and can be specifically modified so as to increase translation of the mRNA.

By "transformation" it is meant the stable interspecific transfer of genetic information that is manifested phenotypically. The constructs of the present invention can be introduced into plant cells using Ti plasmids, Ri plasmids, plant virus vectors, direct DNA transformation, micro-injection, electroporation, etc., as would be known to those of skill in the art. For reviews of such techniques see for example Weissbach and Weissbach, Methods for Plant Molecular Biology, Academy Press, New York VIII, pp. 421-463 (1988); Geierson and Corey, Plant Molecular Biology, 2d Ed. (1988); and Miki and Iyer, Fundamentals of Gene Transfer in Plants. In Plant Metabolism, 2d Ed. DT. Dennis, DH Turpin, DD Lefebrvc, DB Layzell (eds), Addison Wesly, Langmans Ltd. London, pp. 561-579 (1997).

To aid in identification of transformed plant cells, the constructs of this invention may be further manipulated to include plant selectable markers. Useful selectable markers include enzymes that provide for resistance to an antibiotic such as gentamycin, hygromycin, kanamycin, and the like, or enzymes involved in herbicide resistance, for example but not limited to phosphinothricin. Similarly, enzymes providing for production of a compound identifiable by colour change such as GUS ( -glucuronidase), or luminescence, such as GFP or luciferase are useful.

The present invention also pertains to transgenic plants containing a gene construct of the present invention. Methods of regenerating whole plants from plant cells are known in the art, and the method of obtaining transformed and regenerated plants is not critical to this invention. In general, transformed plant cells are cultured in an appropriate medium, which may contain selective agents such as antibiotics, where selectable markers are used to facilitate identification of transformed plant cells. Once callus forms, shoot formation can be encouraged by employing the appropriate plant hormones in accordance with known methods and the shoots transferred to rooting medium for regeneration of plants. The plants may then be used to establish repetitive generations, either from seeds or using vegetative propagation techniques.

The modified EGF of the present invention may be introduced into any desired plant, including forage plants, food crops, or other plants depending upon the need. Examples of such plants include, but not limited to, alfalfa, soybean, wheat, corn, safflower, canola, barley, tobacco, Jerusalem artichoke and potato. In the experiments outlined below, tobacco has been used as the test organism for the expression of the modified EGF, however it is to be understood that the constructs of the present invention may be introduced and expressed in any plant. If desired, the sequence encoding EGF may be further modified for expression within a desired plant using the methods as described herein. For example, the construct comprising the EGF, or a fragment thereof, may also comprise a KDEL sequence, a SAR, a nucleic acid sequence encoding an affinity tag, or a combination thereof, wherein the fragment of EGF exhibits biological activity.

Examples, which are not to be considered limiting, of a modified EGF optimized for expression in canola, comprises the sequences of either SEQ ID NO:26 (Figure 2D; row (5)), or SEQ ID NO: 40 (Figure 2D, row (CS)), however, other plant optimized EGF sequences may be prepared and introduces into a plant of interest, non-food or food crops or forage plants as indicated above. Preferably, the construct comprising the EGF, or a fragment thereof, also comprises a KDEL sequence, a SAR, a nucleic acid sequence encoding an affinity tag, or a combination thereof, wherein the fragment of EGF exhibits biological activity.

The nucleotide sequence of the method of the present invention includes but is not limited to the DNA sequence of a modified EGF as disclosed in SEQ ID NO: 3 and fragments or derivatives thereof, as well as analogues of, or nucleic acid sequences comprising at least about 60.5% similarity with the nucleic acids as defined in SEQ ID NO: 3, and more preferably, at least 70% similarity. The nucleotide sequence of the method of the present invention also includes but is not limited to the DNA sequence of a modified EGF as disclosed in SEQ ID NO's: 23, 24, 26, 27 or 38 to 40, and fragments or derivative thereof, as well as analogues of, or nucleic acid sequences comprising at least about 70% similarity with the nucleic acids as defined in SEQ ID NO:23, 24, 26, 27, or 38 to 40, provided that they exhibit EGF biological activity as previously described.

Analogues include those DNA sequences which hybridize under stringent hybridization conditions, for example, hybridization at 65°C overnight in 0.5 M sodium phosphate, 7% SDS, 10 mM EDTA, salmons sperm DNA, with a wash for 30 min each at 65°C 2xSSC, 0.1% SDS, then 1xSSC, 0.1% SDS, and then 0.1SxSC, 0.1% SDS (see Maniatis et al., in Molecular Cloning , A Laboratory Manual, Cold Spring Harbor Laboratory, 1982, p. 387-389) to any one of the DNA sequences of SEQ ID NO's:3, 11, 26 or 27, provided that said sequences encode an EGF protein that exhibits at least one EGF-biological activity.

Analogues also include nucleic acid sequences exhibiting about an 60,5% homology, more preferably 70% homology, with the sequence defined by any one of SEQ ID NO's:3, 11, 23, 24, 26 or 27, providing that the analogues encode an EGF protein, or a protein exhibiting one or more EGF-biological activities as defined above. Homology between a EGF nucleic acid sequence and an analogue may be readily determined using sequence comparison techniques for example but not limited to a BLAST (available through GenBank URL: www.ncbi.nlm.nih.gov/cgi-bin/BLAST/, using default parameters, including: Program: blastn; Database: nr; Expect 10; filter: low complexity; Alignment: pairwise; Word size: 11) or FASTA, using default parameters However, it is preferred that the nucleotide sequence encodes mature EGF, or a derivative thereof, including EGF KDEL. More preferably the nucleotide sequence encodes mature hEGF or a derivative thereof, including hEGF KDEL.

It is contemplated that a transgenic plant comprising the heterologous protein may be administered to an animal in a variety of ways depending upon the need and the situation For example, if the protein is orally administered, the plant tissue may be harvested and directly feed to the animal, or the harvested tissue may be dried prior to feeding, or the animal may be permitted to graze on the plant with out prior harvest. It is also considered within the scope of this invention for the harvested plant tissues to be provided as a food supplement within animal feed. If the plant tissue is being feed to an animal with little or not further processing it is preferred that the plant tissue being administered is edible. Furthermore, the protein obtained from the transgenic plant may be extracted prior to its use as a food supplement, in either a crude, partially purified, or purified form. In this latter case, the protein may be produced in either edible or non-edible plants.

An example of a plant that is not meant to be limiting in any manner, that can be used for oral administration of the EGF protein of the present invention includes a low alkaloid tobacco (WO/99/67401), for example strain 81V-9. Production of EGF in a low alkaloid tobacco is presented in Example 2 (Figure 3B). However, other edible plants, including food crop, forage, and non-food crop plants may also be used in accordance with the present invention.

Alternatively, the protein produced by the method of the present invention may be partially or completely purified from the plant and reformulated into a desired dosage form. The dosage form may comprise, but is not limited to an oral dosage form wherein the protein is encapsulated, formulated as a solid or gel, or dissolved in a suitable excipient such as but not limited to water. The protein may also be administered via smoke inhalation, as a snuff, or a chewable forms of the leaf, or leaf preparation. In addition, the protein may be formulated into a dosage form that could be applied topically or could be administered by inhaler, or by injection either subcutaneously, into organs, or into circulation. An injectable dosage form may include other carriers that may function to enhance the activity of the protein. The protein produced by the method of the present invention may be formulated for use in the production of a medicament. In this latter case, the protein may be produced in either edible or non-edible plants.

In an embodiment of the method of the present invention, the coding region of the modified EGF may be operatively linked to, for example but not limited to, the alfalfa mosaic virus leader sequence (Genbank accession #V00048 (nt. 1-36); Jobling and Gehrke, 1987, Nature 325:622-625; US 4,820,639), the PR-1b signal sequence (Cornelissen et al. 1986, EMBO J. 5:37-40, Genbank accession #X03465 (nt 30-131)), a scaffold attachment region (Schoff1 et al. 1993, Transgenic Research 2;93-100, Genbank accession # M11317 (nt. 1310-1710)) or a combination thereof, and the fused sequence may be cloned into a vector suitable for expression in a plant, for example, but not limited to pCaMter X (see Examples), comprising a desired regulatory region, for example, but not limited to a tandern 35S CaMV promoter, and a nos terminator, or pCaMter KII comprising 2XCaMV 35S promoter, NOS terminator, and a 3'SAR. In an alternative embodiment, the coding region of the modified EGF may be operatively linked to, which is not to be considered limiting, the alfalfa mosaic virus leader sequence, the PR-1b signal sequence, a KDEL sequence, a SAR, or a combination thereof, for example as described in Example 1, and the fused sequence cloned into a vector suitable for expression in a plant, for example, but not limited to pCaMter X, or pCaMter KII as just described. Nonlimiting examples of constructs comprising the components outlined above include those listed in Table 2A:

Table 2A:

| **listing of several constructs of the present invention comprising SAR and KDEL sequences (also see Figure 1).** | | | | | |
|---|---|---|---|---|---|
| **Name of Construct** | **SAR** | **2x35S-AMV-PR-1b-EGF** | **KDEL** | **NOS** | **SAR** |
| AP.EGF.KDEL.X | --- | √ | √ | √ | --- |
| AP.EGF.KI | √ | √ | --- | √ | √ |
| AP.EGF.KDEL.KI | √ | √ | √ | √ | √ |
| AP.EGF.KII | --- | √ | --- | √ | √ |
| AP.EGF.KDEL.KII | --- | √ | √ | √ | √ |
| AP.EGF.KIII | √ | √ | --- | √ | --- |
| AP.EGF.KDEL.KIII | √ | √ | √ | √ | --- |

A binary vector comprising the cloned genes as outlined above may be introduced into a suitable vector for transformation of a plant, for example but not limited to an *Agrobacterium tumefaciens* strain containing a disarmed Ti plasmid, and plants may be transformed using methods described in the art. However, as one of skill in the art will understand, there exist many other vectors, promoters, terminators and transformation systems which may be used in place of those described herein, for example, but not limited to, pollen transformation, floral dip transformation, or biolistic gene gun transformation as described above. Transformed plants may be determined using any standard methods known in the art for example but not limited to Southern, Northern, or Western analysis, or PCR (see Example 2, Figure 3).

Using the method described herein transformed plants expressing EGF have been produced that express up to about 3.9% of the total soluble protein (see Example 4, Figure 5, construct AP.EGF.KDEL.KI).

Protein encoded by a nucleic acid sequence comprising EOF, for example AP.EGF (or the vector AP.EGF KII, or AP.EGF.X, Figure 1A), comprise the full-length mature EGF protein (53 ammo acids). Nucleotide sequences encoding EGF and KDEL, for example, AP.EGF.KDEL (or the vector AP.EGF KDEL KII, or AP.EGF.KDEL.X Figure 1B), result in a protein product having 4 extra amino acids (Lys, Asp, Glu, Leu; KDEL) at the C-terminal end of the protein, resulting in a 57 amino acid protein. The protein product produced as described herein may be directly administered to a mammal as an oral feed, and does not require further processing as it is produced in its mature form. Both the 53 and 57 amino acid proteins are biologically active in that they are detectable using Western analysis.

The addition of the KDEL (AP.EGF KDEL, AP.EGF.KDEL.KII) sequence results in about a 5 fold to about a 10 fold increase in extractable EGF protein from a plant, when compared to the yields obtained using AP.EGF (AP.EGF.KII). The constructs, plants, and methods of the present inventions produce EGF yields that are up to 650,000-fold higher when compared to the disclosure of Higo et al. (1993, Biosci Biotechnol Biochem 57:1477-1481), and 9,750-fold higher compared with the equivalent mature (6.2 kDa) EGF yields of WO98/21348.

The EGF produced as described herein may be used in a variety of ways including promoting new growth of epithelials cells, for example but not limited to skin, cornea, gastrointestinal tract and lungs. EGF may also be used in wound healing, for example with burn patients, for treatment of surface wounds or multi-organ failure. EGF as produced herein may also be used as a mucosal protectant from oral complications resulting from head and neck radio- or chemotherapy (early evaluation stages), for corneal (eye) wound healing, perforated tympanic membranes (ears), or for treating lung injury. The EGF of the present invention may also be used within diabetes treatment, for example, in treating complication healing (eg. foot ulcer), or pancreatic differentiation and growth. Other uses of the EGF of the present invention include cosmetic skin care products, or use as a veterinary food additive and gastrointestinal therapeutic agent, increased production pigs and beef, a non-antibiotic method to control infection. EGF may also be used for treating premature organ development (e.g. intestine, lungs), or protection of liver from chemical poisoning. EGF is also known to aid in wool gathering from sheep,

For reference purposes, a listing of various EGF sequences of the present invention, which is not to be construed as limiting, is provided in Table 2B, with reference to Figures where they are shown (see Figure legends for more details of the sequences).

Table 2B:

| Sequence Listing Summary | | | | | |
|---|---|---|---|---|---|
| **SEO ID NO:** | **Figure # (row)** | | **SEQ ID NO:** | **Figure # (row)** | |
| SEQ ID NO:1 | 2A | (AA) | SEQ ID NO:23 | 2C | |
| SEQ ID NO:2 | 2A/B | (1) | SEQ ID NO:24 | 2D | (3) |
| SEQ ID NO:3 | 2A | (5) | SEQ ID NO:25 | 2D | (4) |
| SEQ ID NO:11 | 2A | (3) | SEQ ID NO:26 | 2D | (5) |
| SEQ ID NO:12 | 2A | (2) | SEQ ID NO:27 | 2D | (6) |
| SEQ ID NO:13 | 2A | (4) | SEQ ID NO:28 | 2D | (7) |
| SEQ ID NO:17 | 2C | | SEQ ID NO:29 | 2D | (1) |
| SEQ ID NO:18 | 2C | | SEQ ID NO:30 | 2B | (3) |
| SEQ ID NO:19 | 2C | | SEQ ID NO:38 | 2A | (CS) |
| SEQ ID NO:20 | 2C | | SEQ ID NO:39 | 2B | (CS) |
| SEQ ID NO:21 | 2C | | SEQ ID NQ:40 | 2D | (CS) |
| SEQ ID NO:22 | 2C | SEQ ID NO:41 | 2B | (AA) | |

The above description is not intended to limit the claimed invention in any manner, furthermore, the discussed combination of features might not be absolutely necessary for the inventive solution.

The present invention will be further illustrated in the following examples. However it is to be understood that these examples are for illustrative purposes only, and should not be used to limit the scope of the present invention in any manner.

**Example 1**

Synthesis of gene constructs

EGF constructs for transformation into plants were assembled from a series of gene cassettes: AMV-PR, EGF, KDEL, and SAP. The AMV-PR, EGF, and KDEL cassette coding sequences were optimized to reflect codon usage for *N. tabacum*. The constructs comprise components as summarized in Table 3.

Table 3:

| listing of the constructs prepared and assayed in Example 1-4. | | | | | |
|---|---|---|---|---|---|
| **Name of Construct** | **SAR** | **2x35S-AMV-PR-1b-EGF** | **KDEL** | **NOS** | **SAR** |
| AP.EGF.X | --- | √ | --- | √ | --- |
| AP.EGF.KDEL.X | --- | √ | √ | √ | --- |
| AP.EGF.KI | √ | √ | --- | √ | √ |
| AP.EGF.KDEL.KI | √ | √ | √ | √ | √ |
| AP.EGF.KII | --- | √ | --- | √ | √ |
| AP.EGF.KDEL.KII | --- | √ | √ | √ | √ |
| AP.EGF.KIII | √ | √ | --- | √ | --- |
| AP.EGF.KDEL.KIII | √ | √ | √ | √ | --- |

Amino acid sequences for the desired protein products were back-translated to nucleotide sequence using the preferred codons as indicated by the *N.tabacum* codon usage database (www.kazusa.or.jp/codon/cgi- bin/showcodon.cgi?species=Nicotiana+tabacum+[gbpln]). Variation from the preferred codon was done to create or remove restriction enzyme sites and to avoid hairpin loop structures. If two codons showed equal usage, their use was alternated throughout the optimized codon sequence. The following primers were used:

EGF-1s (SEQ ID NO:4),
EGF-2a (SEQ ID NO:5),
EGF-3s (SEQ ID NO:6),
EGF-4a (SEQ ID NO:7),
EGF-Stu1 (SEQ ID NO:8),

as outlined below:

**Primers associated with construction of the EGF and EGF-Stu1 cassettes (amino acid sequence of EGF (SEQ ID NO:1) indicated above the primers):**

```
        HincII
    ValAsnSerAsp SerGluCys ProLeuSer HisAspGlyTyr CysLeuHis

  1 GTTAACTCTG ATTCAGAATG TCCACTTTCT CATG------ ----------  EGF-1s
    ---------- ---------C AGGTGAAAGA GTACTACCAA TAACGGAAGT  EGF-2a

    AspGlyVal CysMetTyrIle GluAlaLeu AspLysTyr AlaCysAsnCys

 51 ---------- ---------- ---------- TGATAAGTAT GCTTGCAATT  EGF-3s
    ACTACCTCAA ACATACATGT AACTTCGAGA ACTATTCATA CGAACG----  EGF-2a

     ValValGly TyrIleGly GluArgCysGln TyrArgAsp LeuLysTrp

101 GTGTTGTTGG TTACATTGGA GAAAGGTGTC AATATAGAGA TCTTAAATGG  EGF-3s
    ---------- ---------- ---------- ---------- --------CC  EGF-4a
                                                 GAATTTACC  EGF-Stu1
                                   BclI
    TrpGluLeuArg End*End* End*End

151 TGGGAGCTTA G--------- ---------- ---  EGF-3s
    ACCCTCGAAT CTATTCATTC ATTCACTAGT GGG  EGF-4a
    ACCCTCGAA* **ATT                      EGF-Stu1*
             /   \
           AGGCCT
           ArgPro
         StuI
```

*Note: EGF-Stu1 primer is used to create a Stu1 restriction site at the 3' end of the EGF cassette. An extra proline amino acid is added but is not maintained after digestion for fusion with the KDEL cassette sequence (see below).

The EGF cassette was constructed from a series of overlapping oligonucleotides (as shown above) designed to encode the mature 53 amino acid active peptide and include $H_{inc}$ II/Hpa 1 and Bcl 1 restriction enzyme sites at the 5' and 3' ends of the cassette respectively. These restriction sites were intended to facilitate addition of upstream regulatory regions and cloning of the assembled gene construct into the plant transformation vector. Melting temperature in the overlap regions between primers varied between 36-44° C. A two-step polymerase chain reaction (PCR) amplification was used to synthesize the EGF cassette: Primers EGF-1s, 2a, 3s, and 4a were mixed in a 1:1 ratio, and initially amplified under low stringency conditions (30 cycles: denature at 95°C for 1 min, anneal at 35°C for 1 min, extend at 75°C for 2 min); a portion of this first reaction was then used as template for PCR under highly stringent conditions (30 cycles: denature at 95°C for I min, anneal at 65°C for 1 min, extend at 75°C for 2 min) using the outside EGF-1 s and EGF-4a primers only to selectively amplify the full-length EGF cassette. VentR® DNA polymerase (New England Biolabs) was used for all PCR amplifications to create blunt ends and allow for editing capability. Amplifications products from the second PCR were cloned into pTZ19U and sequenced to confirm identity.

The AMV-PR cassette was constructed in a similar manner to the EGF cassette using the following overlapping oligonucleotides:

AP bridge (SEQ ID NO:9),
PR-2a (SEQ ID NO:10),
AMV-1s (SEQ ID NO: 33),
PR-1s (SEQ ID NO:34),

as outlined below:

**Primer Design for AMV-PR Cassette (amino acid sequence of EGF (SEQ ID NO:1) indicated above the primers):**

The AMV-PR cassette is designed for insertion into a Sma1-cut cloning vector: On ligation a Sma1 restriction site will be regenerated at the 5' end of the cassette. The 3' end of the cassette incorporates a blunt-cutting Nae1 restriction site and coding for an extra C-terminal glycine amino acid. The glycine residue is effectively removed from coding sequence if the cassette is cut with Nae1 for ligation to the EGF coding sequence. In the AMV/PR primer outlined below, the sequence in italics indicates the AMV-1s primer sequence (SEQ ID NO:33), the sequence in regular text pertains to the PR-1s primer (SEQ ID NO:34):

```
                                                Met GlyPhePhe

  1 GGGTTTTTAT TTTTAATTTT CTTTCAAATA CTTCCATCAT GGGTTTCTTT  AMV/PR
    -------------------------GTTTAT GAAGGTAGTA CCCAAAGAAA  AP bridge

    LeuPheSerGln MetProSer PhePheLeu ValSerThrLeu LeuLeuPhe

 51 CTTTTCTCTC AAATGCCATC ATTTTTCTTG GTTTCTACTT TGC-------  AMV/PR
    GAAAAG----------------------GAAC CAAAGATGAA ACGAAGAAAA  PR-2a

                                         NaeI
     LeuIleIle SerHisSerSer HisAlaGly

101 ---------- ---------- ---------- -
    GAACTAATAA AGTGTAAGAA GTGTACGGCC G     PR-2a
```

The KDEL cassette was constructed by ligation of two complementary primers:

KDEL-1s (SEQ ID NO:31), KDEL-2a (SEQ ID NO:32), as outlined below.

**KDEL Cassette (portion of amino acid sequence of EGF+KDEL (SEQ ID NO:41) indicated above the primers).**

The KDEL cassette includes a 5' DraI restriction site, and a 3' Bcl1 restriction site. Ligation into a Sma1-cut cloning vector further regenerates a Sma1 restriction site at the 3' end of the cassette:

```
           DraI                          BclI
        PheLysAspGlu LeuEnd* End*End*End

      1 TTTAAAGATG AACTTTAAGT AAGTAAGTGA TCACCC         KDEL-1s
        AAATTTCTAC TTGAAATTCA TTCATTCACT AGTGGG         KDEL-2a

                                ATTCA TTCATTCACT AGTGGG         Bc11-term
```

Complementary primers KDEL-1s & 2a form cassette. Note Bc11-term (SEQ ID NO:14) primer also occurs on EGF cassette.

A variation of the EGF cassette carrying a 3' Stu1 restriction site was generated by re-amplifying the EGF cassette with primers EGF-1s and EGF-Stu1. Use of EGF-Stu1 primer results in addition of an extra proline amino acid at the 3' end of the predicted EGF protein, but the proline residue is eliminated after digestion for fusion with the KDEL cassette sequence. The EGF-Stu1 cassette was digested with Stu1, ligated to the Dra1-cut KDEL cassette, and the desired EGF-KDEL cassette generated by PCR amplification using EGF-1s and the Be11-term primers. Cassettes were variously cloned into pTZ and pGEM-T, and sequenced to confirm identity.

AP-EGF and AP-EGF-KDEL cassettes were generated by digestion of the EGF and EGF-KDEL cassettes with HincII, ligation with a NaeI-cut AMV-PR cassette, and PCR amplification of the desired full-length sequences with the AMV-1s and Bell term primers.

**SAR Cassette**

A SAR cassette was amplified by PCR from genomic soybean DNA using specific primers. An example of a SAR from soybean is found in Schoffl et al. (F.Schöffl et al., 1993, Trans. Res. 2, 93-100; Genbank accession M11317, nucleotides 1310-1710). Primers used to amplify SAR are presented below:

**SAR-1s**

5'-GTTAACTAGCAAGTTCAGAGCATC-3' (SEQ ID NO:15)

**SAR-2a**

5'-GGGAATTCTGTCAAAAAAAATATTAAG-3' (SEQ ID NO:16)

The amplified SAR cassette includes unique 5' HpaI/HincII and 3' EcoRI restriction sites. It was amplified using Taq DNA polymerase, subcloned into pGEM-T and sequenced to confirm its identity. The SAR cassette was removed from the cloning vector by digestion with HincII and EcoR1, treated with Klenow to generate blunt-ends, and ligated to a blunt-ended cassette (35S/NOS) consisting of the double 35S promoter and nopaline synthase (NOS) terminator sequence. The 35S/NOS cassette was derived from the empty pCaMter X vector, and included a multiple cloning site. Primers, to the 35S promoter and a modified version of SAR-2a including a 3' Hind III restriction site, was used to selectively amplify correct orientation fusions of the SAR cassette to the 3' end of the 35S/NOS cassette. The resulting 35S/NOS-SAR fusion cassette was subcloned into the pBIN19 backbone to form the pCaMter KII transformation vector.

**Construction of gene constructs X, KI, KII and KIII.**

These primers include restriction sites used in subsequent subcloning of the generated SAR cassette to other genetic elements; a HpaI/HincII restriction site at the extreme 5' end of SAR-1, and an EcoR1 restriction site at the extreme 3' end of SAR-2.

A series of gene constructs were generated incorporating the SAR cassette at various positions relative to the transgene expression cassette. **pCaMter X,** a standard pBIN19-based binary vector containing a gene cassette consisting of the double 35 S promoter and nopaline synthase termination sequence, was used as the base non-SAR vector.

pCaMter X was subjected to Hind III restriction digest and the released element, consisting of the double 35S promoter+NOS terminator expression cassette, was ligated into a pTZ19 plasmid. SAR was variously ligated to the 35S/NOS-pTZ19 construct and the resulting fusion cassettes were subcloned back into a pBIN19 backbone. Final vector constructs consisted of:

**pCaMter KI** which carries a SAR+ double 35S+NOS+SAR cassette,
**pCaMter KII** which carries a double 35S+NOS+SAR cassette, and
**pCaMter KIII** which carries a SAR+double 355+NOS cassette.

All pCaMter vector constructs include right and left T-DNA borders, and an NPT II expression cassette for kanamycin resistance antibiotic selection of transformed plants.

APEGF and APEGFKDEL were ligated into pCaMter series vectors at the BamHI and KpnI restriction sites. These final vector constructs (Figure 1) were sequenced to confirm identity prior to use for plant transformation.

**Example 2**

Transformation of plants

*N.tabacum cv.* Xanthi and a low alkaloid variety, 81V-9, were transformed by *Agrobacterium tumefaciens* infection (Horsch RB, Fry J, Hofmann N, Neidermeyer J, Rogers SG and Fraley RT 1988 Leaf disc transformation Plant Molecular Biology Manual A5/1-A5/9. Kluwer Academic Publishers, Dordrecht/Boston/London.) Plant leaves were sterilized by immersion in a 10% bleach solution for 12-15 min with occasional agitation, rinsed in sterile distilled water and cut to generate leaf discs. *Agrobacterium* cultures were grown to stationary phase under antibiotic selection, and diluted 10-times in sterile MS media for the infection. Leaf discs were swirled into the diluted *Agrobacterium* culture until completely wet, blotted on sterile filter paper, and placed stomata side up on MS shoot-inducing media (MS media/ l mg mL-1 N6-benzyladenine/0,1 mg mL-1 a-naphthalene acetic acid/0.8% agar). Plates were sealed and incubated under a plant growth light at 25°C for 3 days, then transferred onto fresh MS shoot-inducing plates containing kanamycin (300 mg mL-1) and carbenicillin (0.5-1 mg mL-1). Plates were re-scaled and maintained at 25°C for 3-4 weeks until callus was observed to form along the edges of the infected leaf discs. Independent calli, representing separate transformation events, were removed from the discs and transferred onto fresh shoot-inducing plates. Shoots, once formed, were excised from the parent callus and transferred to MS root-inducing media (MS media/0.6% agar) under antibiotic selection (100 mg mL-1 kanamycin and 0.5-1 mg mL-1 carbenicillin). Roots generally formed within 1-3 weeks at which point the regenerated plant was transferred to soil and hardened off to adjust to greenhouse humidity conditions.

Genomic DNA was extracted from transformed plants and transgenic identity confirmed by PCR (Figures 3A and 3B). Quality of the extracted DNA was determined by control amplification of a 475 bp fragment of the tobacco acetolactate synthase gene, a native low-copy number gene. Selective portions of the transgene were also amplified to determine the transgene identity and integration into the plant genome: primers to the CaMV 35S promoter and NOS terminator regions were expected to yield products of approximately 235 bp if plants were transformed with an empty transformation vector, and 550 bp if the desired construct was present; AMV-1s and EGF-4a primers were expected to yield a product of 320 bp; and EGF-1s and a primer to the 3' end of the SAR were expected to yield a product of 780 bp.

**Example 3**

Characterization of protein product

Protein was extracted from young, actively growing leaves at the top half of PCR-identified transgenic plants into 100 mM ammonium bicarbonate buffer (P.Gengenheimer, 1990: Methods of Enzymology 182:1184-185). Total soluble protein (TSP) concentration was estimated by Bradford analysis (M.M.Bradford, 1974, Anal. Biochem. 72: 248-54) using bovine serum albumin as the standard.

Aliquots of total soluble protein extracts from transgenic and wild-type untransformed plants were separated on 5% stacking and 20% separating gels by Tris-glycine SDS-polyacrylamide gel electrophoresis, and transferred to Immuno-blot PVDF membrane (Bio-Rad #162-0177) to identify and determine the size of plant-produced EGF (Figure 4). The resulting Western blots were probed with rabbit polyclonal anti-EGF antibody (Onco-gene Research Products EGF Ab-3, #PC08) followed by goat polyclonal anti-rabbit IgG antibody conjugated with horseradish peroxidase (Oncogene Research Products #DC03L). Detected EGF was visualized by chemiluminescence detection (Amersham Pharmacia). All antibodies were presorbed against total soluble protein extracts from wild-type non-transformed plants prior to use to reduce background detection of plant proteins.

Predicted sizes for the transgene encoded AP-EGF and AP-EGF-KDEL proteins were 9.6 and 10.1 kDa respectively, Western blot analysis showed that the EGF product from AP-EGF plants co-migrated with the mature EGF standard and was slightly smaller than that produced by AP-EGF-KDEL plants: The EGF standard and the plant-produced EGFs were all slightly smaller than a 7.1 kDa molecular weight marker. These results are consistent with the expected 6.2 and 6.7 kDa sizes expected for EGF and EGF-KDEL proteins, and indicate that the 3.4 kDa PR-1b signal peptide is successfully removed from the translated protein within the plant ER. The presence of a soluble, processed EGF protein in plants further provides strong indications that plant-produced EGF will be in active form.

AP.EGF.KDEL constructs appeared to show greater accumulation of protein relative to their AP.EGF counterparts. Similarly, the presence of a SARs sequence also increased protein when compared to contracts lacking SARs.

**Example 4:**

Quantitation of EGF production in transgenic plants.

EGF production was also determined using enzyme-linked immunosorbent assay (ELISA). Mouse monoclonal anti-EGF antibody (Sigma-Aldrich #E2520) was presorbed on 96-well microtitre plates and used to bind EGF present in replicate aliquots of plant protein extracts. Bound EGF was subsequently detected using a rabbit polyclonal anti-EGF antibody (Oncogene Research Products EGF Ab-3, #PC08) and a polyclonal goat anti-rabbit IgG antibody conjugated with alkaline phosphatase (Oncogene research products DC06L). All polyclonal antibodies were presorbed against total protein extracts from untransformed plants to reduce background detection of plant proteins.

Quantitation of results was based on p-nitrophenyl phosphate disodium (pNPP, Sigma Aldrich) oxidation by the alkaline peroxidase, detected at 405 nm. This method allowed for simultaneous analysis of a large number of samples and estimation of EGF content based a standard curve (0-200 ng EGF: Gibco/BRL #13247-051). Final EGF production by a given plant was calculated as a percentage of the total soluble protein present: [ELISA estimate of amount EGF (ng/uL) *100]/[Bradford estimate amount total soluble protein (ng/uL)].

Amounts of EGF produced by transformed plants ranged from 0.006-3.9% of total soluble protein (Figure 5). For this analysis 38 AP.EGF X plants, 29 AP.EGF KDEL X plants, 12 AP.EGF K1 plants and 36 AP.EGF.KDEL K1 plants, were used. Statistical analysis (GLM Procedure of SAS: SAS Institute, Kary N.Carolina) found a significant difference in the amount of EGF present in plants carrying the AP.EGF KI vs. AP.EGF X constructs indicating that the presence of the SAR enabled greater accumulation of EGF. AP.EGF.KDEL constructs also tended to show greater accumulation compared to AP.EGF constructs as previously suggested by Western blots analysis. Highest levels of expression were seen in AP.EGF.KDEL KI transgenic plants. No difference in EGF accumulation was seen relating to the tobacco cultivar used,

The ELISA estimates of EGF production in plants as descried above (0.006-3.9%) demonstrate a substantial increase in the levels of EGF, over those reported in prior art of about a1000-650,000 fold increase, when compared to Higo *et al*. (1993, BioSci Biotech Biochem 57:1477-1481) who report 0.0000006% production of EGF, based on ELISA estimates, and about 15-9,750 fold increase when compared to Hooker *et al*. (WO 98/21348) who report 0.0004% production of EGF, again based on ELISA estimates.

All citations are herein incorporated by reference.

The present invention has been described with regard to preferred embodiments. However, it will be obvious to persons skilled in the art that a number of variations and modifications can be made without departing from the scope of the invention as described herein.

## Claims

1. A nucleic acid molecule that encodes an epidermal growth factor protein (EGF) or a fragment thereof, the nucleic acid molecule also comprising a KDEL sequence, a scaffold attachment region (SAR), a nucleic acid sequence encoding an affinity tag, or a combination thereof, wherein the fragment of EGF exhibits biological activity.

2. The nucleotide sequence of claim 1, wherein the EGF has been optimized for expression in plants.

3. The nucleic acid molecule defined in claim 2, wherein the EGF is hEGF.

4. The nucleic acid molecule defined in claim 3, wherein the hEGF is encoded by the nucleotide sequence defined by SEQ ID NO:3, an analogue, fragment, or derivative thereof, providing that the analogue, fragment, or derivative thereof encodes a product that exhibits EGF-biological activity, the analogue, fragment, or derivative thereof comprising at least about 60.5% homology with the nucleotide sequence defined by SEQ ID NO:3 as determined using BLAST, with the following . parameters: Program: blastn; Database: nr; Expect 10; filter: low complexity; Alignment: pairwise; Word size: 11.

5. The nucleic acid molecule defined in claim 3, wherein the hEGF is encoded by the nucleotide sequence defined by SEQ ID NO:3, an analogue, fragment, or derivative thereof, providing that the analogue, fragment, or derivative thereof encodes a product that exhibits EGF-biological activity, the analogue, fragment, or derivative thereof hybridizes to the hEGF under stringent conditions, the stringent conditions comprising, hybridization at 65°C overnight in 0.5 M sodium phosphate, 7% SDS, 10 mM EDTA, salmons sperm DNA, followed by washing, for 30 min each, at 65°C 2xSSC, 0.1% SDS, then 1xSSC, 0.1% SDS, and then 0.1SxSC, 0.1% SDS.

6. The nucleic acid molecule as defined by claim 1 further comprising at least one nucleotide sequence encoding a signal sequence peptide operatively linked with the modified nucleotide sequence encoding the EGF.

7. The nucleic acid molecule as defined by claim 6, wherein the at least one nucleotide sequence encoding a signal sequence peptide is obtained from a protein selected from the group consisting of a pathogenesis related protein, pathogenesis-related protein 1a, pathogenesis-related protein 1b, pathogenesis-related protein 1c, pathogenesis-related protein S, sporamin, extensin, potato proteinase inhibitor II, lectin, EGF, preproricin, human alpha-lattalbumin, and human alpha-lactoferrin.

8. The nucleic acid molecule as defined by claim 1, wherein the scaffold attachment region is selected from the group consisting of a soybean, a tobacco, a tomato, an *Arabidopsis*, and a petunia .

9. The nucleic acid molecule defined in claim 1, wherein the nucleic acid molecule is AP.EGF.

**10.** The nucleic acid molecule defined in claim 1, wherein the nucleic acid molecule is AP.EGF.KDEL.

**11.** A vector comprising the nucleic acid molecule of claim 1, operatively linked with a regulatory region and terminator region.

**12.** A vector comprising the nucleic acid molecule of claim 2, operatively linked with a regulatory region and terminator region.

**13.** A vector comprising the nucleic acid molecule of claim 3, operatively linked with a regulatory region and terminator region.

**14.** A vector comprising the nucleic acid molecule of claim 9.

**15.** A vector comprising the nucleic acid molecule of claim 10.

**16.** A plant cell, plant seed, a plant, or progeny thereof, comprising the vector of claim 11.

**17.** A plant cell, plant seed, a plant, or progeny thereof, comprising the vector of claim 12.

**18.** A plant cell, plant seed, a plant, or progeny thereof, comprising the vector of claim 13.

**19.** A plant cell, plant seed, a plant, or progeny thereof, comprising the vector of claim 14.

**20.** A plant cell, plant seed, a plant, or progeny thereof, comprising the vector of claim 15.

**21.** A method of producing a transgenic plant that expresses an epidermal growth factor comprising;

i) introducing into a plant, the nucleic acid molecule of claim 1 to produce one or more transformed plants;
ii) selecting from the one or more transformed plants an EGF-expressing transformed plant; and
iii) growing the EGF-expressing transformed plant to produce the transgenic plant that expresses EGF.

**22.** A method of treating a mammal in need of epidermal growth factor (EGF) comprising,

i) introducing into a plant, the nucleic acid molecule of claim 1 to produce one or more transformed plants;
ii) selecting from the one or more transformed plants an EGF-expressing transformed plant;
iii) growing the EGF-cxpressing transformed plant to produce a transgenic plant that expresses EGF;
iv) feeding the transgenic plant that expresses EGF to the mammal.

**23.** A method for producing epidermal growth factor (EGF) comprising,

i) introducing into a plant, the nucleic acid molecule of claim 1 to produce one or more transformed plants;
ii) selecting from the one or more transformed plants an EGF-expressing transformed plant;
iii) growing the EGF-expressing transformed plant to produce a transgenic plant that expresses EGF;
iv) harvesting tissue from the transgenic plant that expresses EGF; and
v) extracting the EGF from the tissue.

**24.** The method of claim 23 wherein, following the step of extracting, the EGF is purified.

**25.** A method of producing an epidermal growth factor comprising, growing the plant of claim 16 to produce the EGF.

**26.** A method of treating a mammal in need of epidermal growth factor (EGF) comprising, growing the plant of claim 16 to produce the EGF, and feeding the plant, or an extract therefrom, to the mammal.

**27.** The nucleic acid molecule defined in claim 2, wherein the EGF is selected from the group consisting of hEGF, pig EGF, rat EGF, mouse EGF, cat EGF, dog EGF and horse EGF.

**28.** The nucleic acid molecule defined in claim 27, wherein the EGF is cat EGF.

**29.** The nucleic acid molecule defined in claim 27, wherein the cat EGF is encoded by the nucleotide sequence defined by SEQ ID NO:23, an analogue, fragment, or derivative thereof, providing that the analogue, fragment, or derivative thereof encodes a product that exhibits EGF-biological activity, the analogue, fragment, or derivative thereof comprising at least about 70% homology with the nucleotide sequence defined by SEQ ID NO:3 as determined using BLAST, with the following parameters: Program: blastn; Database: nr; Expect 10; filter: low complexity; Alignment: pairwise; Word size: 11.

Figure 1A
AP.EGF.KII

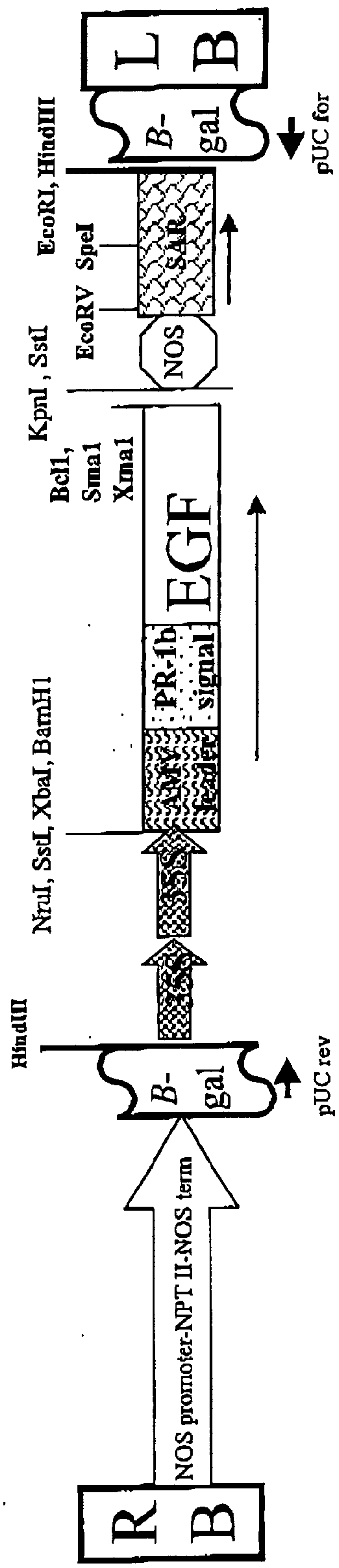


Figure 1B
AP.EGF.KDEL.KII

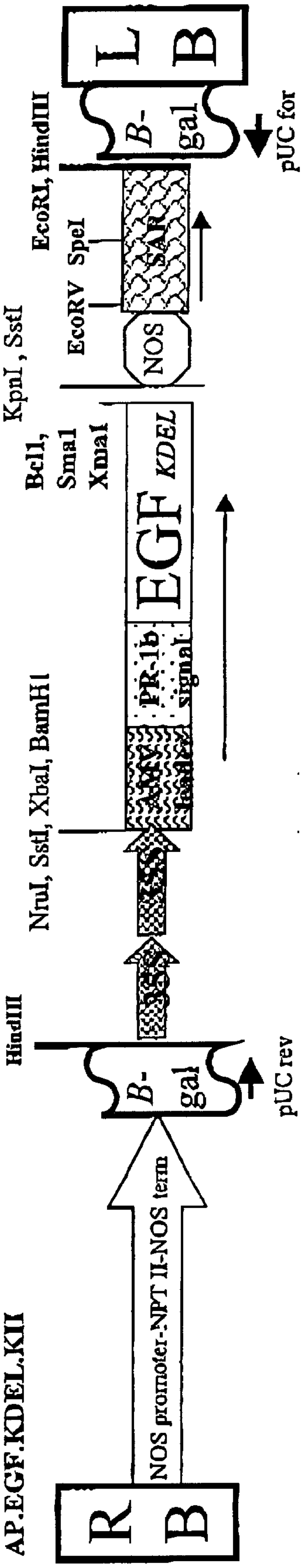


Figure 1

Figure 1C
AP.EGF X

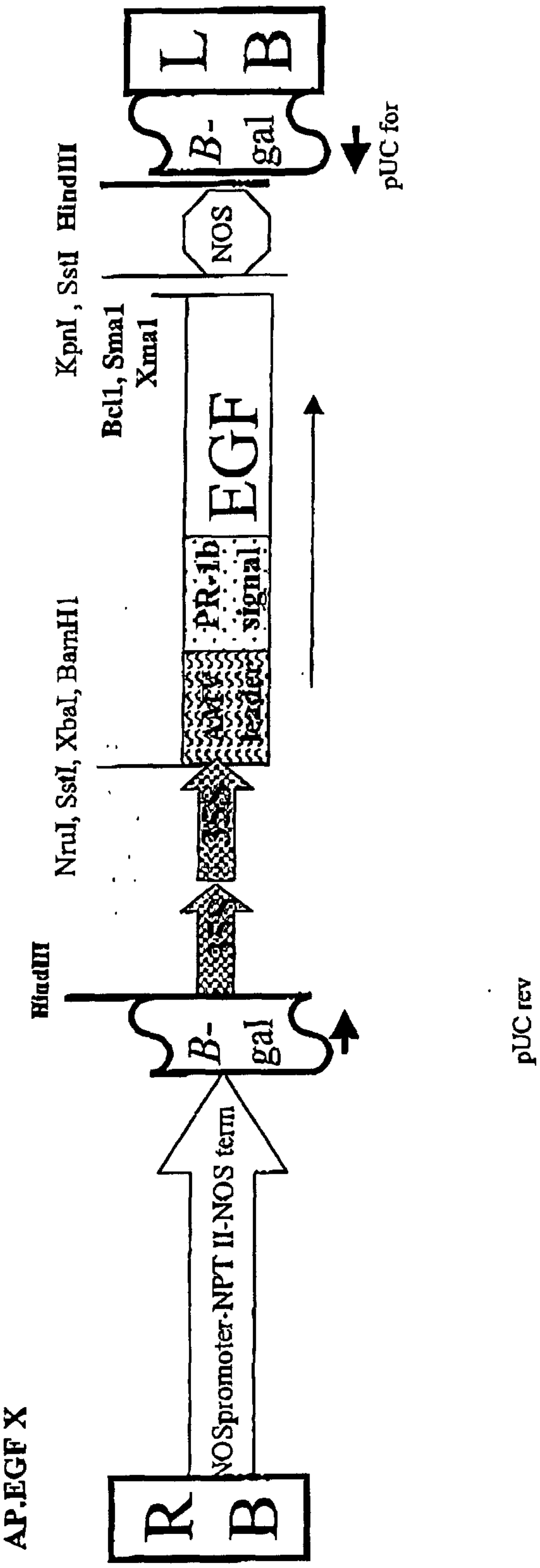


Figure 1D
AP.EGF.KDEL X

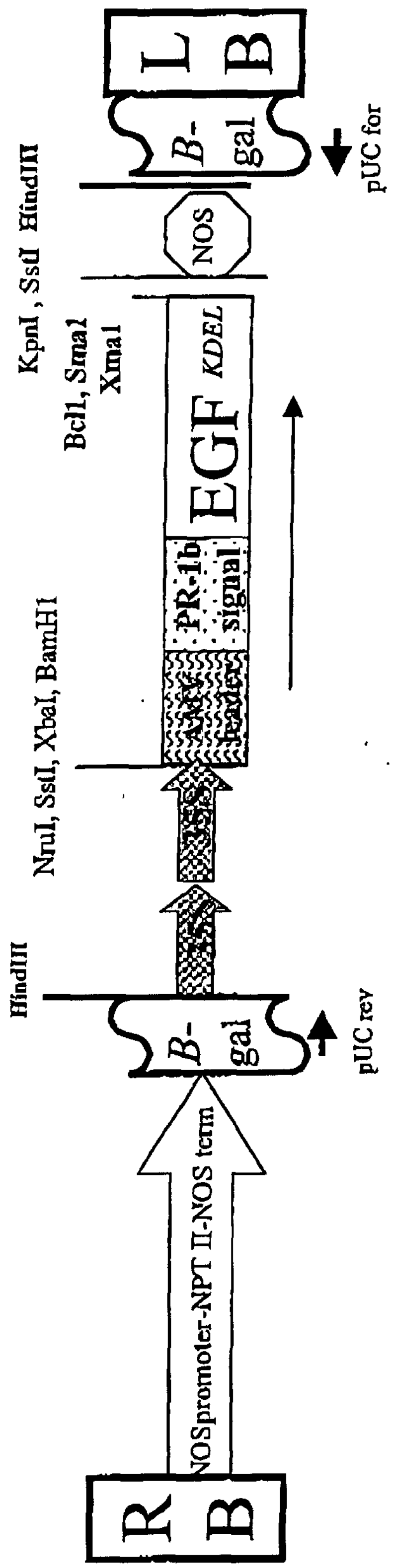


Figure 1 continued

**Figure 1E**
AP.EGF KI

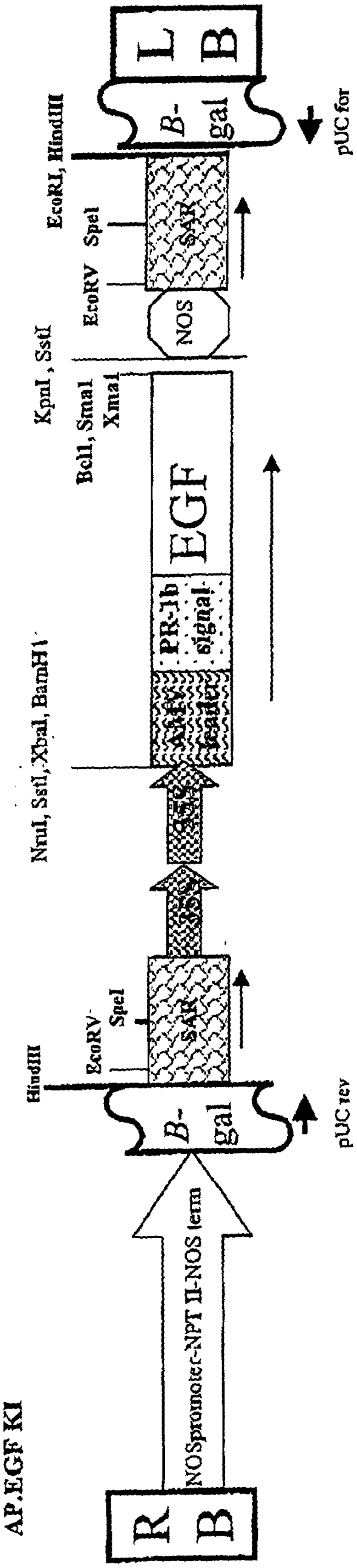


**Figure 1F**
AP.EGF.KDEL KI

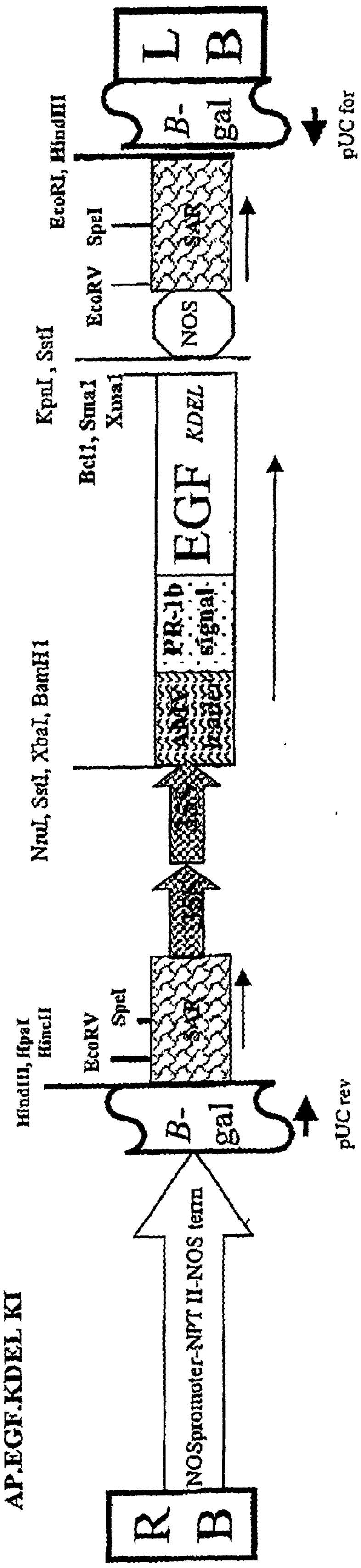


Figure 1 continued

Figure 1G
AP.EGF KIII

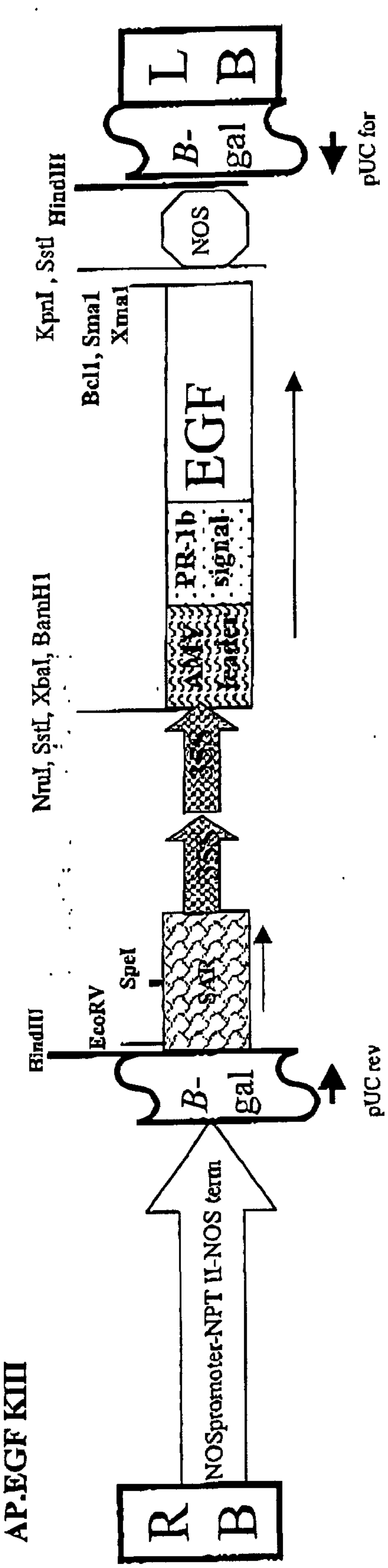


Figure 1H
AP.EGF.KDEL KIII

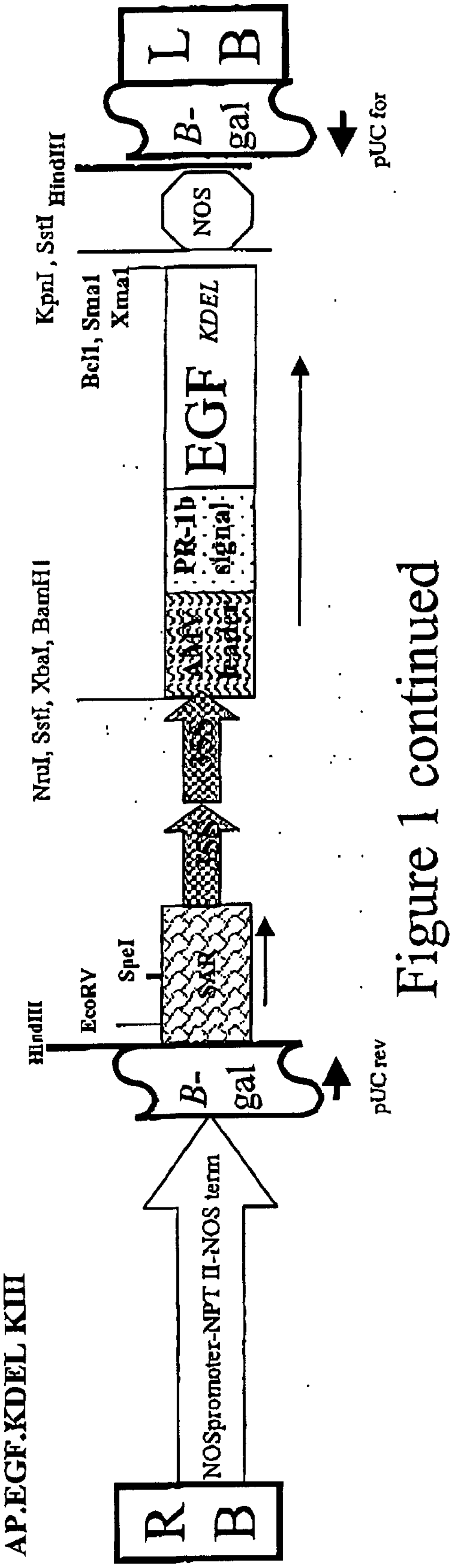


Figure 1 continued

**Comparison of Nucleotide Sequences Encoding hEGF for Expression in Tobacco**

```
AsnSerAspSerGluCysProLeuSerHisAspGlyTyrCysLeuHisAspGlyValCys  (AA)
AAywsnGAywsnGArTGyCCnyTdwsbCAyGAyGGnTAyTGyyTnCAyGAyGGnGTnTGy  (CS)

AATAGTGACTCTGAATGTCCCCTGTCCCACGATGGGTACTGCCTCCATGATGGTGTGTGC  (1)
AACTCVGATAGCGAGTGCCCDTTAAGTCATGACGGHTATTGTTTRCACGACGGVGTHTGT  (2)

AATTCTGATTCTGAATGTCCACTTTCTCATGATGGTTATTGTCTTCATGATGGTGTTTGT  (3)
AACTCGGACTCGGAGTGCCCGCTATCGCACGACGGGTACTGCCTACACGACGGGGTATGC  (4)
AACTCTGATTCAGAATGTCCACTTTCTCATGATGGTTATTGCCTTCATGATGGAGTTTGT  (5)


MetTyrIleGluAlaLeuAspLysTyrAlaCysAsnCysValValGlyTyrIleGlyGlu  (AA)
ATGTAyAThGArGCnyTnGAyAArTAyGCnTGyAAyTGyGTnGTnGGnTAyAThGGdGAr  (CS)

ATGTATATTGAAGCATTGGACAAGTATGCATGCAACTGTGTTGTTGGCTACATCGGGGAG  (1)
ATGTACATMGAGGCBCTHGATAAATACGCBTGTAATTGCGTVGTVGGDTATATWGGGGAG  (2)

ATGTATATTGAAGCTCTTGATAAGTATGCTTGTAATTGTGTTGTTGGTTATATTGGTGAA  (3)
ATGTACATAGAGGCGCTAGACAAATACGCGTGCAACTGCGTAGTAGGGTACATCGGGGAG  (4)
ATGTACATTGAAGCTCTTGATAAGTATGCTTGCAATTGTGTTGTTGGTTACATTGGAGAA  (5)


ArgCysGlnTyrArgAspLeuLysTrpTrpGluLeuArgEnd*End*End*End        (AA)
mGrTGyCArTAymGrGAyyTdAArTGGTGGGAryTdmGvTrr                    (CS)

CGATGTCAGTACCGAGACCTGAAGTGGTGGGAACTGCGCTGA                    (1)
AGGTGCCAGTATAGGGATTTAAAATGGTGGGAGTTAAGRTGR                    (2)

AGATGTCAATATAGAGATCTTAAGTGGTGGGAACTTAGATAA                    (3)
CGGTGCCAGTACCGGGACCTAAAATGGTGGGAGCTACGGTAG                    (4)
AGGTGTCAATATAGAGATCTTAAATGGTGGGAGCTTAGATAAGTAAGTAAGTGATCACC   (5)
```

**FIGURE 2A**

**Comparison of various hEGF Sequences for Expression in Tobacco**

```
AsnSerAspSerGluCysProLeuSerHisAspGlyTyrCysLeuHisAspGlyValCys (AA)

AAywsTGAyTCwGAATGTCCmCTkTCyCAyGATGGkTAyTGCCTyCATGATGGwGTkTGy (CS)

AATAGTGACTCTGAATGTCCCCTGTCCCACGATGGGTACTGCCTCCATGATGGTGTGTGC (1)
AACTCTGATTCAGAATGTCCACTTTCTCATGATGGTTATTGCCTTCATGATGGAGTTTGT (2)
AACTCTGATTCAGAATGTCCACTTTCTCATGATGGTTATTGCCTTCATGATGGAGTTTGT (3)


MetTyrIleGluAlaLeuAspLysTyrAlaCysAsnCysValValGlyTyrIleGlyGlu (AA)

ATGTAyATTGAAGCwyTkGAyAAGTATGCwTGCAAyTGTGTTGTTGGyTACATyGGrGAr (CS)

ATGTATATTGAAGCATTGGACAAGTATGCATGCAACTGTGTTGTTGGCTACATCGGGGAG (1)
ATGTACATTGAAGCTCTTGATAAGTATGCTTGCAATTGTGTTGTTGGTTACATTGGAGAA (2)
ATGTACATTGAAGCTCTTGATAAGTATGCTTGCAATTGTGTTGTTGGTTACATTGGAGAA (3)


ArgCysGlnTyrArgAspLeuLysTrpTrpGluLeuArgLysAspGluLeuEnd*End*  (AA)

mGrTGTCArTAymGAGAyCTkAArTGGTGGGArCTkmGmaaagatgaacttTrAGTAAG  (CS)

CGATGTCAGTACCGAGACCTGAAGTGGTGGGAACTGCGC------------tga-----  (1)
AGGTGTCAATATAGAGATCTTAAATGGTGGGAGCTTAGA-----------TAAGTAAG  (2)
AGGTGTCAATATAGAGATCTTAAATGGTGGGAGCTTAGAAAAGATGAACTTTAAGTAAG  (3)


End*End                                                      (AA)

TAAGTGATCACC                                                 (CS)

-----------                                                  (1)
TAAGTGATCACC                                                 (2)
TAAGTGATCACC                                                 (3)
```

FIGURE 2B

**EGF Homology Across Species**

| | | | | | | |
|---|---|---|---|---|---|---|
| | 1 | | | | | 53 |
| EGF (A) | NSDSECPLSH | DGYCLHDGVC | MYIEALDKYA | CNCVVGYIGE | RCQYRDLKWW | ELR |
| Human | NSDSECPLSH | DGYCLHDGVC | MYIEALDKYA | CNCVVGYIGE | RCQYRDLKWW | ELR |
| Pig | NSYSECPPSH | DGYCLHGGVC | MYIEAVDSYA | CNCVFGYVGE | RCQHRDLKWW | ELR |
| Rat | NSNTGCPPSY | DGYCLNGGVC | MYVESVDRYV | CNCVIGYIGE | RCQHRDLRWW | KLR |
| Mouse | NSYPGCPSSY | DGYCLNGGVC | MHIESLDSYT | CNCVIGYSGD | RCQTRDLRWW | ELR |
| Cat | NSYQECPPSY | DGYCLYNGVC | MYIEAVDRYA | CNCVFGYVGE | RCQHRDLKW- | ELR |
| Dog | NGYRECPSSY | DGYCLYNGVC | MYIEAVDRYA | CNCVFGYVGE | RCQHRDLKW- | ELR |
| Horse | NSYQECSQSY | DGYCLHGGKC | VYLVQVDTHA | CNCVVGYVGE | RCQHQDLR | |
| Consensus | NS--ECP-S+ | DGYCL+-GVC | MYIE++D+YA | CNCV-GY+GE | RCQ+RD-+W- | ELR |

**FIGURE 2C**

## Cat mature EGF nucleotide sequence for expression in tobacco and canola

**AsnSerTyrGlnGluCysProProSerTyrAspGlyTyrCysLeuTyrAsnGlyValCys (AA)**
AAywskTAyCArGArTGyCCnCCnTCnTAyGAyGGdTAyTGyCThTAyAAyGGdGTdTGy (CS)

AACAGTTACCAGGAATGCCCCCCATCCTATGACGGGTACTGCCTCTATAACGGTGTGTGT (1)

AATTCTTATCAAGAATGTCCACCATCTTATGATGGTTATTGTCTTTATAATGGTGTTTGT (2)
AATTCTTATCAAGAATGTCCTCCATCATATGATGGATATTGTCTTTACAATGGTGTTTGC (3)
AACTCGTACCAGGAGTGCCCGCCGTCGTACGACGGGTACTGCCTATACAACGGGGTATGC (4)

AACTCTTACCAGGAGTGCCCTCCTTCTTACGATGGATACTGCCTTTACAACGGAGTTTGC (5)
AATTCGTATCAAGAATGTCCCCCCTCGTATGACGGGTATTGTCTATATAATGGGGTATGT (6)
AACTCTTATCAGGAATGTCCTCCATCTTACGATGGATACTGTCTCTACAATGGTGTGTGC (7)

**MetTyrIleGluAlaValAspArgTyrAlaCysAsnCysValPheGlyTyrValGlyGlu (AA)**
ATGTAyATdGArGCdGTdGAymGrTAyGCdTGyAAyTGyGTdTTyGGnTAyGTwGGdGAr (CS)

ATGTATATTGAAGCAGTGGACAGATACGCATGCAACTGTGTTTTTGGCTATGTTGGTGCG (1)

ATGTATATTGAAGCTGTTGATAGATATGCTTGTAATTGTGTTTTTGGTTATGTTGGTGAA (2)
ATGTACATTGAGGCTGTTGACAGGTATGCTTGTAACTGCGTGTTTGGTTACGTTGGAGAG (3)
ATGTACATAGAGGCGGTAGACCGGTACGCGTGCAACTGCGTATTCGGGTACGTAGGGGAG (4)

ATGTACATCGAGGCTGTTGATAGATACGCTTGCAACTGCGTTTTTGGATACGTTGGAGAG (5)
ATGTATATAGAAGCGGTAGACCGGTATGCGTGTAATTGTGTATTCGGGTATGTAGGGGAA (6)
ATGTACATCGAAGCTGTTGACAGGTATGCTTGCAACTGCGTGTTTGGATATGTTGGTGAG (7)

**ArgCysGlnHisArgAspLeuLysTrpGluLeuArg (AA)**
vGrTGbCArCAhvGdGAbsTwAAdTGGGArbTdmG (CS)

GGAGCGATGTCAGCACCGGGACTTGAAATGGGAAC (1)

AGATGTCAACATAGAGATCTTAAGTGGGAACTTAG (2)
AGGTGCCAACATAGAGATCTTAAGTGGGAATTGAG (3)
CGGTGCCAGCACCGGGACCTAAAATGGGAGCTACG (4)

AGATGCCAGCATAGAGATCTTAAGTGGGAGCTTAG (5)
CGGTGTCAACACCGGGACCTAAAATGGGAACTACG (6)
AGATGTCAACATCGTGATCTTAAGTGGGAGCTTAG (7)

# FIGURE 2D

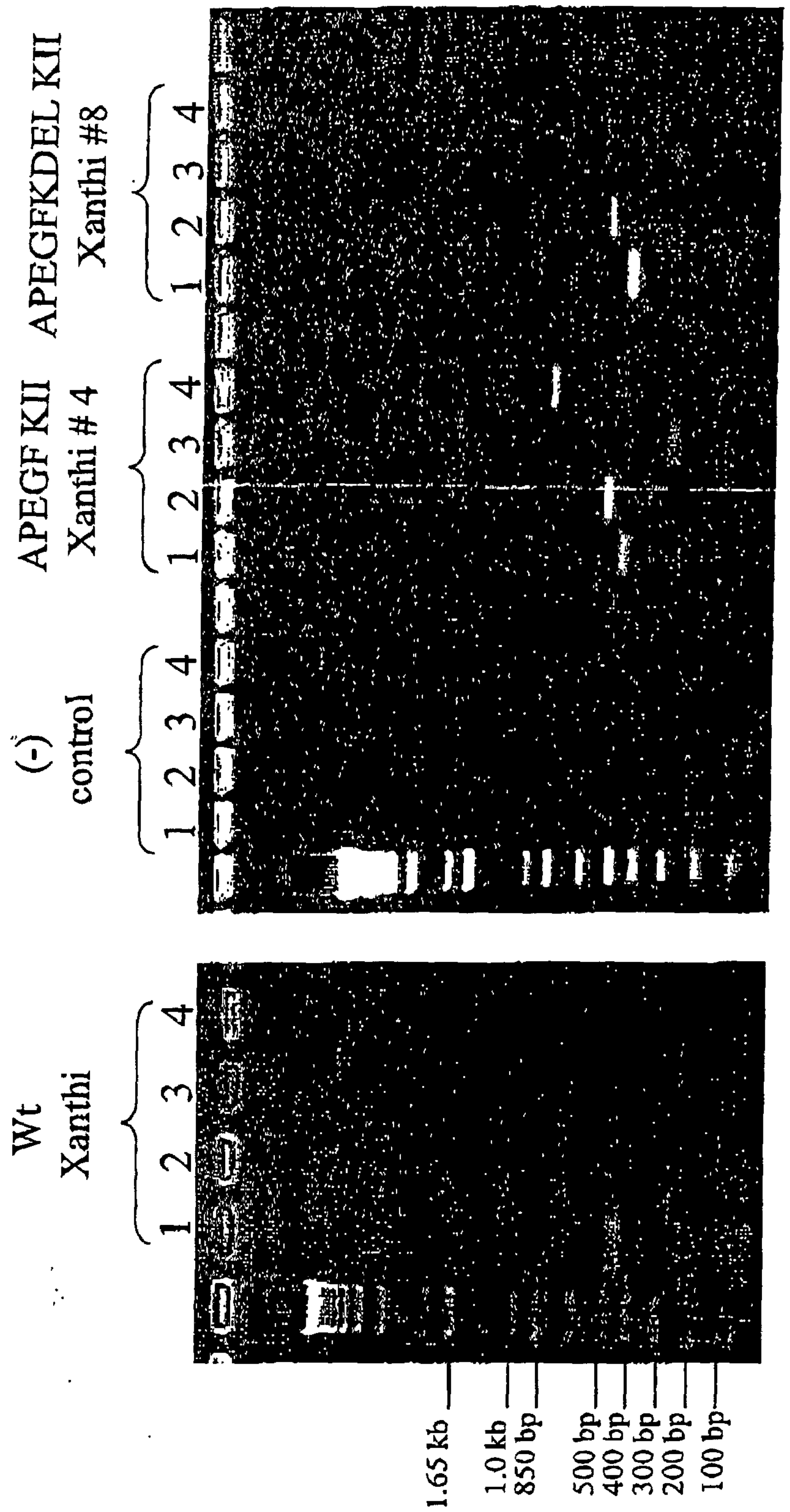
Wt
Xanthi
1 2 3 4
(-)
control
1 2 3 4
APEGF KII
Xanthi # 4
1 2 3 4
APEGFKDEL KII
Xanthi #8
1 2 3 4
1.65 kb
1.0 kb
850 bp
500 bp
400 bp
300 bp
200 bp
100 bp

Figure 3A

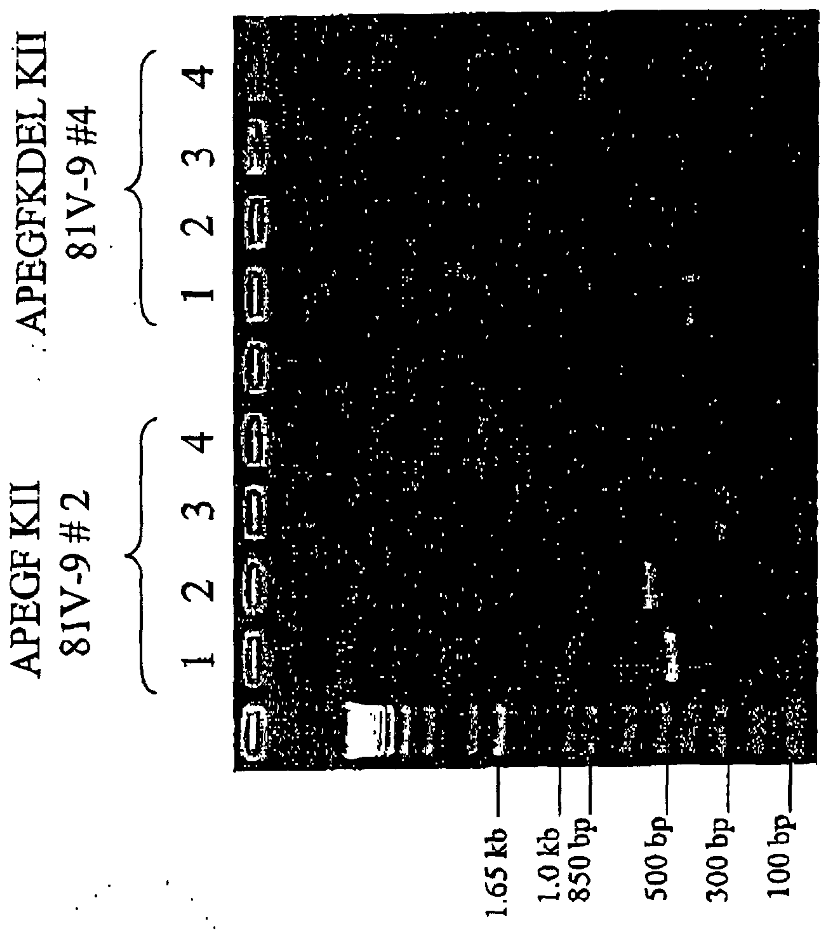
APEGF KII
81V-9 # 2
1 2 3 4
APEGFKDEL KII
81V-9 #4
1 2 3 4
1.65 kb
1.0 kb
850 bp
500 bp
300 bp
100 bp

Figure 3B

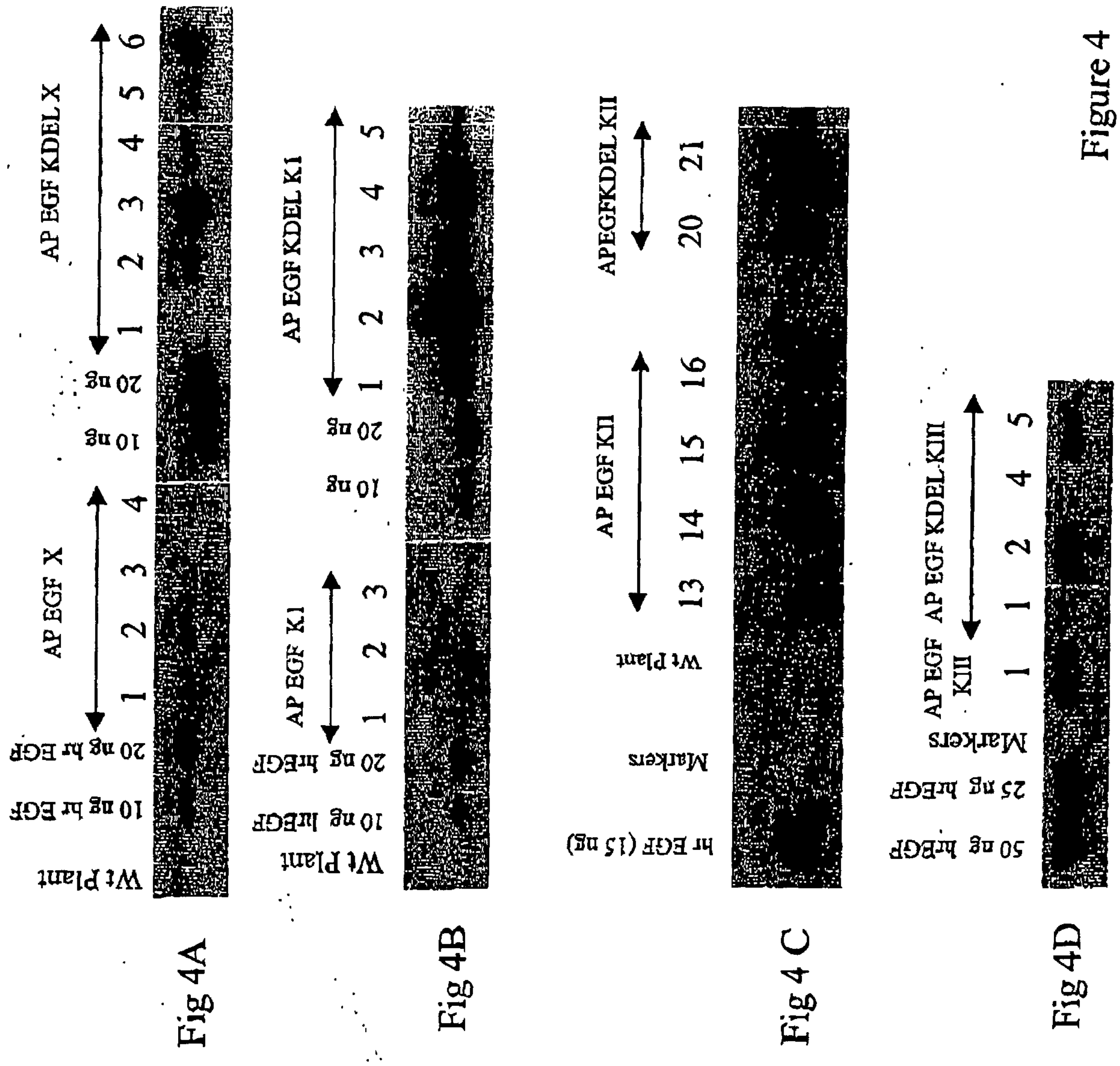
Fig 4A
Wt Plant
10 ng hr EGF
20 ng hr EGF
AP EGF X
1 2 3 4
10 ng
20 ng
AP EGF KDEL X
1 2 3 4 5 6
Fig 4B
Wt Plant
10 ng hrEGF
20 ng hrEGF
AP EGF K1
1 2 3
10 ng
20 ng
AP EGF KDEL K1
1 2 3 4 5
Fig 4 C
hr EGF (15 ng)
Markers
Wt Plant
AP EGF KII
13 14 15 16
APEGFKDEL KII
20 21
Fig 4D
50 ng hrEGF
25 ng hrEGF
Markers
AP EGF KII
AP EGF KDEL KII
1 1 2 4 5

Figure 4

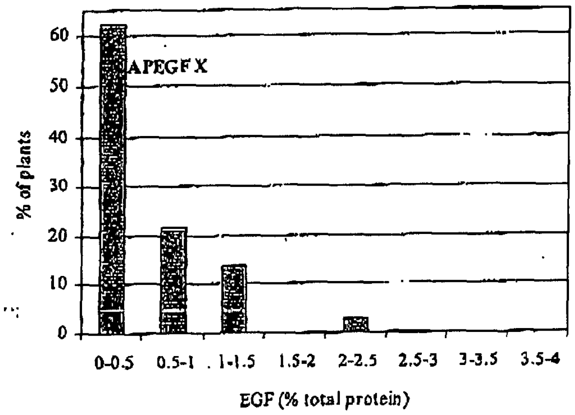


Figure 5A

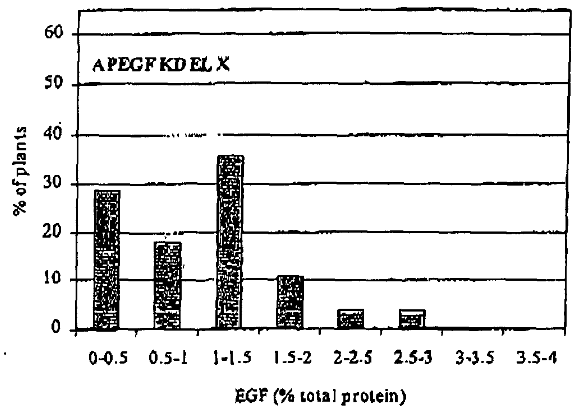


Figure 5B

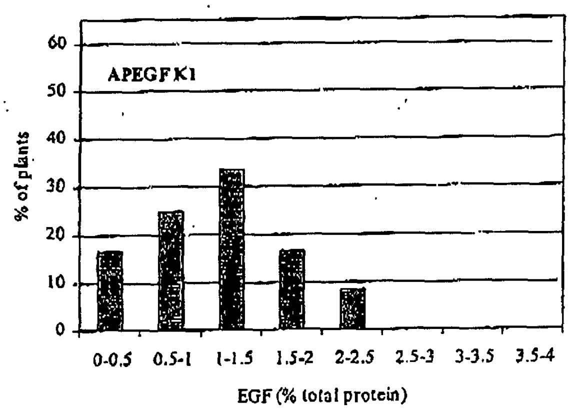


Figure 5C

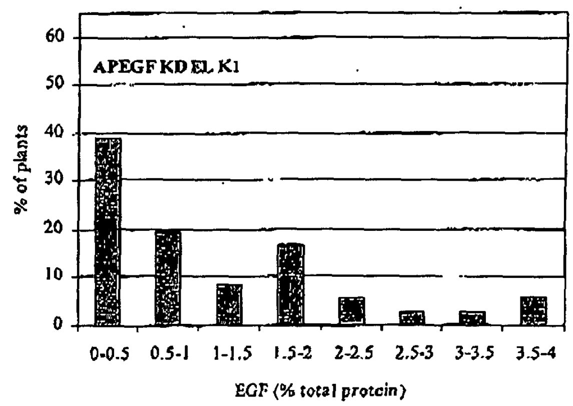


Figure 5D

SEQUENCE LISTING

<110> Alberta Research Council of Canada

<120> Production of Recombinant Epidermal Growth Factor in Plants

<130> 08-892965US

<150> US 60/377,294

<151> 2002-04-29

<160> 41

<170> PatentIn version 3.0

<210> 1

<211> 53

<212> PRT

<213> homo sapien

<400> 1

Asn Ser Asp Ser Glu Cys Pro Leu Ser His Asp Gly Tyr Cys Leu His
1 5 10 15

Asp Gly Val Cys Met Tyr Ile Glu Ala Leu Asp Lys Tyr Ala Cys Asn
20 25 30

Cys Val Val Gly Tyr Ile Gly Glu Arg Cys Gln Tyr Arg Asp Leu Lys
35 40 45

Trp Trp Glu Leu Arg
50

<210> 2

<211> 162

<212> DNA

<213> homo sapien

<400> 2
aatagtgact ctgaatgtcc cctgtcccac gatgggtact gcctccatga tggtgtgtgt 60

atgtatattg aagcattgga caagtatgca tgcaactgtg ttgttggcta catcggggag 120

cgatgtcagt accgagacct gaagtggtgg gaactgcgct ga 162

<210> 3

<211> 179

<212> DNA

<213> homo sapien

<400> 3
aactctgatt cagaatgtcc actttctcat gatggttatt gcctccatga tggagtttct 60

atgtacattg aagctcttga caagtatgct tgcaattgtg ttgttggtta cattggagaa 120

aggtgtcaat atagagatct caaatggtgg gagcttagat aagtaagtaa gtgatcacc 179

<210> 4

<211> 34

<212> DNA

<213> artificial

<400> 4
gttaactctg attcagaatg tccactttct catg 34

<210> 5

<211> 77

<212> DNA

<213> artificial

<400> 5
caggtgaaag agcactacca ataacggaag tactacctca aacatacatg taacttccag 60

aactattcat acgaacg 77

<210> 6

<211> 81

<212> DNA

<213> artificial

<400> 6
tgataagtat gcttgcaatt gtgttgttgg ttacattgga gaaaggtgtc aatatagaga 60

tcttaaatgg tgggagctta g 81

<210> 7

<211> 35

<212> DNA

<213> artificial;

<400> 7
ccacccctcga atctattcat tcattcacta gtggg 35

<210> 8

<211> 27

<212> DNA

<213> artificial

<400> 8
gaatttacca ccctcgaaag gcctatt 27

<210> 9

<211> 32

<212> DNA

<213> artificial

<400> 9
gtttatgaag gtagtaccca aagaaagaaa ag 32

<210> 10

<211> 55

<212> DNA

<213> artificial

<400> 10
gaaccaaaga tgaaacgaag aaaagaacta ataaagtgta agaagtgtac ggccg 55

<210> 11

<211> 162

<212> DNA

<213> artificial

<400> 11
aattctgatt ctgaatgtcc actttctcat gatggttatt gtcttcatga tggtgtttgt 60

atgtatattg aagctcttga taagtatgct tgtaattgtg ttgctggtta tattggtgaa 120

agatgtcaat atagagatct taagtggtgg gaacttagat aa 162

<210> 12

<211> 162

<212> DNA

<213> artificial

<400> 12
aactcvgata gcgagtgccc dttaagtcat gacgghtatt gtttrcacga cggvgthtgt 60

atgtacatwg aggcbctbga taaatacgcb tgtaattgcg tvgcvggdta tatwggggag 120

aggtgccagt atagggattt aaaatggtgg gagttaagtt gt 162

<210> 13

<211> 162

<212> DNA

<213> artificial

<400> 13
aactcggact cggagtgccc gctatcgcac gacgggtact gcctacacga cggggtatgc 60

atgtacatag aggcgctaga caaatacgcg tgcaaatgcg tagtagggta catcggggag 120

cggtgccagt accgggacct aaaatggtgg gagctacggt ag 162

<210> 14

<211> 21

<212> DNA

<213> artificial

<400> 14
atccattcat tcactagtgg g 21

<210> 15

<211> 24

<212> DNA

<213> artificial

<400> 15
gttaaccagc aagttcagag catc 24

<210> 16

<211> 27

<212> DNA

<213> artificial

<400> 16
gggaattctg tcaaaaaaaa tattaag 27

<210> 17

<211> 53

<212> PRT

<213> sus scrofa

<400> 17

Asn Ser Tyr Ser Glu Cys Pro Pro Ser His Asp Gly Tyr Cys Leu His
1 5 10 15

Gly Gly Val Cys Met Tyr Ile Glu Ala Val Asp Ser Tyr Ala Cys Asn
20 25 30

Cys Val Phe Gly Tyr Val Gly Glu Arg Cys Gln His Arg Asp Leu Lys
35 40 45

Trp Trp Glu Leu Arg
50

<210> 18

<211> 53

<212> PRT

<213> rattus norvegicus

<400> 18

Asn Ser Asn Thr Gly Cys Pro Pro Ser Tyr Asp Gly Tyr Cys Leu Asn
1 5 10 15

Gly Gly Val Cys Met Tyr Val Glu Ser Val Asp Arg Tyr Val Cys Asn
20 25 30

Cys Val Ile Gly Tyr Ile Gly Glu Arg Cys Gln His Arg Asp Leu Arg
35 40 45

Trp Trp Lys Leu Arg
50

<210> 19

<211> 53

<212> PRT

<213> mus musculus

<400> 19

Asn Ser Tyr Pro Gly Cys Pro Ser Ser Tyr Asp Gly Tyr Cys Leu Asn
1 5 10 15

Gly Gly Val Cys Met His Ile Glu Ser Leu Asp Ser Tyr Thr Cys Asn
20 25 30

Cys Val Ile Gly Tyr Ser Gly Asp Arg Cys Gln Thr Arg Asp Leu Arg
35 40 45

Trp Trp Glu Leu Arg
50

<210> 20

<211> 52

<212> PRT

<213> felis catus

<400> 20

Asn Ser Tyr Gln Glu Cys Pro Pro Ser Tyr Asp Gly Tyr Cys Leu Tyr
1 5 10 15

Asn Gly Val Cys Met Tyr Ile Glu Ala Val Asp Arg Tyr Ala Cys Asn
20 25 30

Cys Val Phe Gly Tyr Val Gly Glu Arg Cys Gln His Arg Asp Leu Lys
35 40 45

Trp Glu Leu Arg
50

<210> 21

<211> 52

<212> PRT

<213> canis familiaris

<400> 21

Asn Gly Tyr Arg Glu Cys Pro Ser Ser Tyr Asp Gly Tyr Cys Leu Tyr
1 5 10 15

Asn Gly Val Cys Met Tyr Ile Glu Ala Val Asp Arg Tyr Ala Cys Asn
20 25 30

Cys Val Phe Gly Tyr Val Gly Glu Arg Cys Gln His Arg Asp Leu Lys
35 40 45

Trp Glu Leu Arg
50

<210> 22

<211> 48

<212> PRT

<213> equus caballus

<400> 22

Asn Ser Tyr Gln Glu Cys Ser Gln Ser Tyr Asp Gly Tyr Cys Leu His
1 5 10 15

Gly Gly Lys Cys Val Tyr Leu Val Gln Val Asp Thr His Ala Cys Asn
20 25 30

Cys Val Val Gly Tyr Val Gly Glu Arg Cys Gln His Gln Asp Leu Arg
35 40 45

<210> 23

<211> 155

<212> DNA

<213> artificial

<400> 23
aattcttatc aagaacgtcc accatcttac gatggttatt gtctttataa tggtgtttgt 60

atgtacattg aagctgttga tagatatgcc cgtaattgcg tttttggtta tgttggtgaa 120

agatgtcaac atagagatct taagtgggaa cttag 155

<210> 24

<211> 155

<212> DNA

<213> artificial

<400> 24
aattcttatc aagaacgtcc tccatcatat gatggatatt gtctttacaa tggtgtttgc 60

atgtacattg aggctgttga caggtatgcc tgtaactgcg tgtttggtta cgttggagag 120

aggtgccaac atagagatct taagtgggaa ttgag 155

<210> 25

<211> 155

<212> DNA

<213> artificial

<400> 25
aactcgtacc aggagtgccc gccgtcgtac gacgggtact gcctatacaa cggggtatgc 60

atgtacatag aggcggtaga ccggtacgcg tgcaactgcg tattcgggta cgtaggggag 120

cggtgccagc accgggacct aaaatgggag ctacg 155

<210> 26

<211> 155

<212> DNA

<213> artificial

<400> 26
aactcttacc aggagtgccc tccttcttac gatggatact gcctttacaa cggagtttgc 60

atgtacatcg aggctgttga tagatacgcc tgcaactgcg tttttggata cgttggagag 120

agatgccagc atagagatct taagtgggag cttag 165

<210> 27

<211> 155

<212> DNA

<213> artificial

<400> 27
aattcgtatc aagaatgtcc cccctcgtat gacgggtatt gtctatataa tggggtatgt 60

atgtatatag aagcggtaga ccggtatgcg tgtaattgtg tattcgggta tgtaggggaa 120

cggtgtcaac accgggaccc aaaatgggaa ctacg 155

<210> 28

<211> 155

<212> DNA

<213> artificial

<400> 28
aactcttatc aggaatgtcc tccatcttac gatggatact gtctctacaa tggtgtgtgc 60

atgtacatcg aagctgttga caggtatgct tgcaactgcg tgtttggata tgttggcgtg 120

agatgtcaac atcgtgatct taagtgggag cttag 155

<210> 29

<211> 155

<212> DNA

<213> artificial

<400> 29
aacagttacc aggaatgccc cccatcctat gacgggtact gcctctataa cggtgtgtgt 60

atgtatattg aagcagtgga cagatacgca tgcaactgtg tttttggcta tgttggtgcg 120

ggagcgatgt cagcaccggg acttgaaatg ggaac 155

<210> 30

<211> 191

<212> DNA

<213> artificial

<400> 30

aactctgatt caggatgtcc actttctcat gatggttatt gccttcatga tggagtttg 60

atgtacattg aagctcttga taagtatgct tgcaattgtg ttgttggtta gattggagaa 120

aggtgtcaat atagagatct taaatggtgg gagcttagaa aagatgaact ttaagtaag 180

aagtgatcac c 191

<210> 31

<211> 36

<212> DNA

<213> artificial

<400> 31
tttaaagatg aactttaagt aagtaagtga tcaccc 36

<210> 32

<211> 36

<212> DNA

<213> artificial

<400> 32
aatttctac ttgaaattca ttcattcact agtggg 36

<210> 33

<211> 38

<212> DNA

<213> artificial

<400> 33
gggttttcat ttttaatttt gtttcaaata cttccatc 38

<210> 34

<211> 55

<212> DNA

<213> artificial

<400> 34
atgggttctt ttctttttctc tcaaatgcca tcattttttct tggtttctac tttgc 55

<210> 35

<211> 10

<212> DNA

<213> artificial

<400> 35
aataaamaaa 10

<210> 36

<211> 10

<212> DNA

<213> artificial

<400> 36
ttwtattwtt 10

<210> 37

<211> 15

<212> DNA

<213> artificial

<220>

<221> n

<222> (3)..(3)

<223> where "n" stands for a or t or g or c

<220>

<221> n

<222> (10)..(10)

<223> where "n" stands for a or t or g or c

<220>

<221> n

<222> (13)..(13)

<223> where "n" stands for a or t or g or c

<220>

<221> n

<222> (14)..(14)

<223> where "n" stands for a or t or g or c

<400> 37
gtnwayattn atnnr 15

<210> 38

<211> 162

<212> DNA

<213> artificial

<220>

<221> n

<222> (6)..(6)

<223> where "n" stands for a or t or g or c

<220>

<221> n

<222> (21)..(21)

<223> where "n" stands for a or t or g or c

<220>

<221> n

<222> (36)..(36)

<223> where "n" stands for a or t or g or c

<220>

<221> n

<222> (45)..(45)

<223> where "n" stands for a or t or g or c

<220>

<221> n

<222> (54)..(54)

<223> where "n" stands for a or t or g or c

<220>

<221> n

<222> (57)..(57)

<223> where "n" stands for a or t or g or c

<220>

<221> n

<222> (75)..(75)

<223> where "n" stands for a or t or g or c

<220>

<221> n

<222> (78)..(78)

<223> where "n" stands for a or t or g or c

<220>

<221> n

<222> (90)..(90)

<223> where "n" stands for a or t or g or c

<220>

<221> n

<222> (12)..(12)

<223> where "n" stands for a or t or g or c

<220>

<221> n

<222> (101)..(101)

<223> where "n" stands for a or t or g or c

<220>

<221> n

<222> (104)..(104)

<223> where "n" stands for a or t or g or c

<220>

<221> n

<222> (107)..(107)

<223> where "n" stands for a or t or g or c

<400> 38
aaywsngayw sngartgycc nytdwsbcay gayggncayt gyytncayga yggngtntgy 60

atgtayathg argcnytnga yaartaygcn tgyaaytgyg tngtnggnta yathggdgay 120

mgrtgycart aymgrgayyt daartggtgg garytdmgvt rr 162

<210> 39

<211> 191

<212> DNA

<213> artificial

<400> 39
aaywstgayt cwgaatgtcc mctktcycay gatggktayt gcctycatga tggwgtktgy 60

atgtayattg aagcwytkga yaagtatgcw tgcaaytgtg ttgttggyta catyggrgay 120

mgrtgtcart aymgagayct kaartggtgg garctkmgma aagatgaact ttragtaagt 180

aagtgatcac c 191

<210> 40

<211> 155

<212> DNA

<213> artificial

<220>

<221> n

<222> (21)..(21)

<223> where "n" stands for a or t or g or c

<220>

<221> n

<222> (24)..(24)

<223> where "n" stands for a or t or g or c

<220>

<221> n

<222> (27)..(27)

<223> where "n" stands for a or t or g or c

<220>

<221> n

<222> (108)..(108)

<223> where "n" stands for a or t or g or c

<400> 40
aaywskrayc argartgycc nccnccntay gayggdrayr gycthrayaa yggdgtdtgy 60

atgtayatdg argcdgtdga ymgrtaygcd tgyaaytgyg tdttyggnta ygtwggdgar 120

vgrtgbcarc ahvgdgabst waadtgggar btdmg 155

<210> 41

<211> 57

<212> PRT

<213> homo sapien

<400> 41

```
Asn Ser Asp Ser Glu Cys Pro Leu Ser His Asp Gly Tyr Cys Leu His
1               5                   10                  15

Asp Gly Val Cys Met Tyr Ile Glu Ala Leu Asp Lys Tyr Ala Cys Asn
            20                  25                  30

Cys Val Val Gly Tyr Ile Gly Glu Arg Cys Gln Tyr Arg Asp Leu Lys
        35                  40                  45

Trp Trp Glu Leu Arg Lys Asp Glu Leu
    50                  55
```